(19) Europäisches Patentamt
European Patent Office
Office européen des brevets

(11) EP 2 546 263 A2

(12) **EUROPEAN PATENT APPLICATION**
published in accordance with Art. 153(4) EPC

(43) Date of publication:
**16.01.2013 Bulletin 2013/03**

(21) Application number: **11713459.3**

(22) Date of filing: **11.03.2011**

(51) Int Cl.:
***C07K 14/435*** (2006.01) ***C12N 15/12*** (2006.01)
***C12N 15/63*** (2006.01) ***D01F 4/00*** (2006.01)

(86) International application number:
**PCT/KR2011/001730**

(87) International publication number:
**WO 2011/112046 (15.09.2011 Gazette 2011/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2010 KR 20100021934**

(71) Applicant: **Korea Advanced Institute of Science and Technology**
**Daejeon-si 305-701 (KR)**

(72) Inventors:
- **LEE, Sang Yup**
  **Daejeon 305-761 (KR)**
- **XIA, Xiaoxia**
  **Daejeon 305-701 (KR)**
- **QIAN, Zhi Gang**
  **Daejeon 305-701 (KR)**
- **LEE, Jeong Wook**
  **Daejeon 305-701 (KR)**
- **PARK, Young Hwan**
  **Seoul 151-921 (KR)**

(74) Representative: **Hiebl, Inge Elisabeth**
**Kraus & Weisert**
**Patent- und Rechtsanwälte**
**Thomas-Wimmer-Ring 15**
**80539 München (DE)**

# (54) HIGH MOLECULAR WEIGHT RECOMBINANT SILK OR SILK-LIKE PROTEIN, AND MICRO- OR NANOSCALE SPIDER-WEB FIBER OR SPIDER-WEB-LIKE FIBER PRODUCED USING THE RECOMBINANT SILK OR SILK-LIKE PROTEIN

(57) The present invention relates to a high-molecular-weight recombinant silk or silk-like protein having a molecular weight which is substantially similar to that of native silk protein, and to a micro- or nano-sized spider silk or silk-like fiber having improved physical properties, produced therefrom. The recombinant silk or silk-like protein according to the invention has high molecular weight, like dragline silk proteins from spiders, while a fiber produced therefrom has excellent physical properties compared to a fiber produced from native silk protein. Thus, the recombinant silk or silk-like protein and the spider silk or silk-like fiber produced therefrom will be highly useful in various industrial applications, including bioengineering applications and medical applications.

FIG. 4

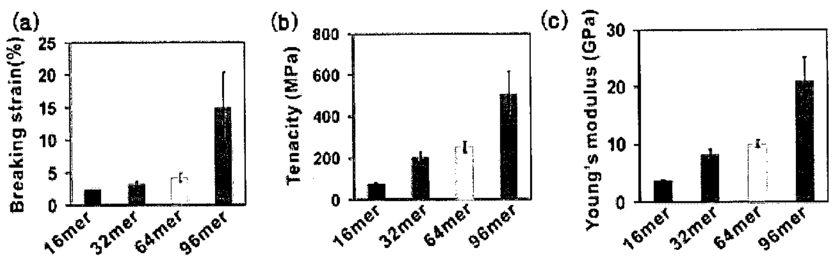




Printed by Jouve, 75001 PARIS (FR)

SEQUENCE LISTING

```
<110>  KOREA ADVANCED INSTITUTE OF SCIENCE AND TECHNOLOGY

<120>  HIGH-MOLECULAR-WEIGHT RECOMBINANT SILK OR SILK-LIKE PROTEIN AND
       MICRO- OR NANO-SIZED SPIDER SILK OR SILK-LIKE FIBER PRODUCED THEREFROM

<130>  22652EP

<140>  PCT/KR2011/001730
<141>  2011-03-11

<150>  KR 10-2010-0021934
<151>  2010-03-11

<160>  22

<170>  PatentIn version 3.2

<210>  1
<211>  35
<212>  PRT
<213>  Artificial

<220>
<223>  Repeat unit of Dragline silk protein

<400>  1

Ser Gly Arg Gly Gly Leu Gly Gly Gln Gly Ala Gly Met Ala Ala Ala
1               5                   10                  15


Ala Ala Met Gly Gly Ala Gly Gln Gly Gly Tyr Gly Gly Leu Gly Ser
            20                  25                  30


Gln Gly Thr
        35


<210>  2
<211>  5
<212>  PRT
<213>  Artificial

<220>
<223>  Repeat unit of Dragline silk protein

<400>  2

Gly Pro Gly Gln Gln
1               5


<210>  3
<211>  5
<212>  PRT
```

<213>  Artificial

<220>
<223>  Repeat unit of Dragline silk protein

<400>  3

Gly Pro Gly Gly Tyr
1               5

<210>  4
<211>  7
<212>  PRT
<213>  Artificial

<220>
<223>  Repeat unit of Dragline silk protein

<400>  4

Gly Gly Tyr Gly Pro Gly Ser
1               5

<210>  5
<211>  5
<212>  PRT
<213>  Artificial

<220>
<223>  Repeat unit of elastin

<400>  5

Gly Val Gly Val Pro
1               5

<210>  6
<211>  4
<212>  PRT
<213>  Artificial

<220>
<223>  Repeat unit of elastin

<400>  6

Val Pro Gly Gly
1

<210>  7
<211>  6
<212>  PRT
<213>  Artificial

<220>
<223>  Repeat unit of elastin

<400>  7

Ala Pro Gly Val Gly Val
1               5

<210>  8
<211>  6
<212>  PRT
<213>  Artificial

<220>
<223>  Repeat unit of silk fibroin

<400>  8

Gly Ala Gly Ala Gly Ser
1               5

<210>  9
<211>  5
<212>  PRT
<213>  Artificial

<220>
<223>  Repeat unit of byssus

<400>  9

Gly Pro Gly Gly Gly
1               5

<210>  10
<211>  5
<212>  PRT
<213>  Artificial

<220>
<223>  Repeat unit of flagelliform silk

<220>
<221>  misc_feature
<222>  (5)..(5)
<223>  Xaa can be any naturally occurring amino acid

<400>  10

Gly Pro Gly Gly Xaa
1               5

<210>  11

<211>  15
<212>  PRT
<213>  Artificial

<220>
<223>  Repeat unit of collagen

<400>  11

Gly Ala Pro Gly Ala Pro Gly Ser Gln Gly Ala Pro Gly Leu Gln
1               5                   10                  15

<210>  12
<211>  15
<212>  PRT
<213>  Artificial

<220>
<223>  Repeat unit of collagen

<400>  12

Gly Ala Pro Gly Thr Pro Gly Pro Gln Gly Leu Pro Gly Ser Pro
1               5                   10                  15

<210>  13
<211>  7117
<212>  DNA
<213>  Artificial

<220>
<223>  pSH16a

<400>  13
tggcgaatgg gacgcgccct gtagcggcgc attaagcgcg gcgggtgtgg tggttacgcg     60

cagcgtgacc gctacacttg ccagcgccct agcgcccgct cctttcgctt tcttcccttc    120

ctttctcgcc acgttcgccg gctttccccg tcaagctcta aatcgggggc tccctttagg    180

gttccgattt agtgctttac ggcacctcga ccccaaaaaa cttgattagg gtgatggttc    240

acgtagtggg ccatcgccct gatagacggt ttttcgccct ttgacgttgg agtccacgtt    300

ctttaatagt ggactcttgt tccaaactgg aacaacactc aaccctatct cggtctattc    360

ttttgattta taagggattt tgccgatttc ggcctattgg ttaaaaaatg agctgattta    420

acaaaaattt aacgcgaatt ttaacaaaat attaacgttt acaatttcag gtggcacttt    480

tcggggaaat gtgcgcggaa cccctatttg tttatttttc taaatacatt caaatatgta    540

tccgctcatg aattaattct tagaaaaact catcgagcat caaatgaaac tgcaatttat    600

tcatatcagg attatcaata ccatattttt gaaaaagccg tttctgtaat gaaggagaaa    660

actcaccgag gcagttccat aggatggcaa gatcctggta tcggtctgcg attccgactc 720
gtccaacatc aatacaacct attaatttcc cctcgtcaaa aataaggtta tcaagtgaga 780
aatcaccatg agtgacgact gaatccggtg agaatggcaa aagtttatgc atttctttcc 840
agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400

ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700
ggtcactgat gcctccgtgt aagggggatt tctgttcatg ggggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatgtcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140

ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatcga tctcgatccc 4980
gcgaaattaa tacgactcac tataggggaa ttgtgagcgg ataacaattc ccctctagaa 5040
ataattttgt ttaactttaa gaaggagata tacatatgca ccatcatcat catcattctt 5100
ctggtctggt gccacgcggt tctggtatga aagaaaccgc tgctgctaaa ttcgaacgcc 5160
agcacatgga cagcccagat ctgggtaccg acgacgacga caaggccatg gctgatatcg 5220
gatccatggc tagcggtcgc ggcggtctgg gtggccaggg tgcaggtatg gcggctgcgg 5280
ctgcaatggg cggtgctggc caaggtggct acggcggcct gggttctcag ggtactagcg 5340
gtcgcggcgg tctgggtggc cagggtgcag gtatggcggc tgcggctgca atgggcggtg 5400
ctggccaagg tggctacggc ggcctgggtt ctcagggtac tagcggtcgc ggcggtctgg 5460
gtggccaggg tgcaggtatg gcggctgcgg ctgcaatggg cggtgctggc caaggtggct 5520
acggcggcct gggttctcag ggtactagcg gtcgcggcgg tctgggtggc cagggtgcag 5580
gtatggcggc tgcggctgca atgggcggtg ctggccaagg tggctacggc ggcctgggtt 5640
ctcagggtac tagcggtcgc ggcggtctgg gtggccaggg tgcaggtatg gcggctgcgg 5700
ctgcaatggg cggtgctggc caaggtggct acggcggcct gggttctcag ggtactagcg 5760
gtcgcggcgg tctgggtggc cagggtgcag gtatggcggc tgcggctgca atgggcggtg 5820
ctggccaagg tggctacggc ggcctgggtt ctcagggtac tagcggtcgc ggcggtctgg 5880

gtggccaggg tgcaggtatg gcggctgcgg ctgcaatggg cggtgctggc caaggtggct 5940

acggcggcct gggttctcag ggtactagcg gtcgcggcgg tctgggtggc cagggtgcag 6000

gtatggcggc tgcggctgca atgggcggtg ctggccaagg tggctacggc ggcctgggtt 6060

ctcagggtac tagcggtcgc ggcggtctgg gtggccaggg tgcaggtatg gcggctgcgg 6120

ctgcaatggg cggtgctggc caaggtggct acggcggcct gggttctcag ggtactagcg 6180

gtcgcggcgg tctgggtggc cagggtgcag gtatggcggc tgcggctgca atgggcggtg 6240

ctggccaagg tggctacggc ggcctgggtt ctcagggtac tagcggtcgc ggcggtctgg 6300

gtggccaggg tgcaggtatg gcggctgcgg ctgcaatggg cggtgctggc caaggtggct 6360

acggcggcct gggttctcag ggtactagcg gtcgcggcgg tctgggtggc cagggtgcag 6420

gtatggcggc tgcggctgca atgggcggtg ctggccaagg tggctacggc ggcctgggtt 6480

ctcagggtac tagcggtcgc ggcggtctgg gtggccaggg tgcaggtatg gcggctgcgg 6540

ctgcaatggg cggtgctggc caaggtggct acggcggcct gggttctcag ggtactagcg 6600

gtcgcggcgg tctgggtggc cagggtgcag gtatggcggc tgcggctgca atgggcggtg 6660

ctggccaagg tggctacggc ggcctgggtt ctcagggtac tagcggtcgc ggcggtctgg 6720

gtggccaggg tgcaggtatg gcggctgcgg ctgcaatggg cggtgctggc caaggtggct 6780

acggcggcct gggttctcag ggtactagcg gtcgcggcgg tctgggtggc cagggtgcag 6840

gtatggcggc tgcggctgca atgggcggtg ctggccaagg tggctacggc ggcctgggtt 6900

ctcagggtac tagtggatcc gaattcgagc tccgtcgaca agcttgcggc cgcactcgag 6960

caccaccacc accaccactg agatccggct gctaacaaag cccgaaagga agctgagttg 7020

gctgctgcca ccgctgagca ataactagca taaccccttg gggcctctaa acgggtcttg 7080

aggggttttt tgctgaaagg aggaactata tccggat 7117

<210> 14
<211> 3600
<212> DNA
<213> Artificial

<220>
<223> 32mer silk protein nucleic acid sequence

<400> 14
atgcaccatc atcatcatca ttcttctggt ctggtgccac gcggttctgg tatgaaagaa 60

accgctgctg ctaaattcga acgccagcac atggacagcc cagatctggg taccgacgac 120

gacgacaagg ccatggctga tatcggatcc atggctagcg gtcgcggcgg tctgggtggc 180

cagggtgcag gtatggcggc tgcggctgca atgggcggtg ctggccaagg tggctacggc 240
ggcctgggtt ctcagggtac tagcggtcgc ggcggtctgg gtggccaggg tgcaggtatg 300
gcggctgcgg ctgcaatggg cggtgctggc caaggtggct acggcggcct gggttctcag 360
ggtactagcg gtcgcggcgg tctgggtggc cagggtgcag gtatggcggc tgcggctgca 420
atgggcggtg ctggccaagg tggctacggc ggcctgggtt ctcagggtac tagcggtcgc 480
ggcggtctgg gtggccaggg tgcaggtatg gcggctgcgg ctgcaatggg cggtgctggc 540
caaggtggct acggcggcct gggttctcag ggtactagcg gtcgcggcgg tctgggtggc 600
cagggtgcag gtatggcggc tgcggctgca atgggcggtg ctggccaagg tggctacggc 660
ggcctgggtt ctcagggtac tagcggtcgc ggcggtctgg gtggccaggg tgcaggtatg 720
gcggctgcgg ctgcaatggg cggtgctggc caaggtggct acggcggcct gggttctcag 780
ggtactagcg gtcgcggcgg tctgggtggc cagggtgcag gtatggcggc tgcggctgca 840
atgggcggtg ctggccaagg tggctacggc ggcctgggtt ctcagggtac tagcggtcgc 900
ggcggtctgg gtggccaggg tgcaggtatg gcggctgcgg ctgcaatggg cggtgctggc 960
caaggtggct acggcggcct gggttctcag ggtactagcg gtcgcggcgg tctgggtggc 1020
cagggtgcag gtatggcggc tgcggctgca atgggcggtg ctggccaagg tggctacggc 1080
ggcctgggtt ctcagggtac tagcggtcgc ggcggtctgg gtggccaggg tgcaggtatg 1140
gcggctgcgg ctgcaatggg cggtgctggc caaggtggct acggcggcct gggttctcag 1200
ggtactagcg gtcgcggcgg tctgggtggc cagggtgcag gtatggcggc tgcggctgca 1260
atgggcggtg ctggccaagg tggctacggc ggcctgggtt ctcagggtac tagcggtcgc 1320
ggcggtctgg gtggccaggg tgcaggtatg gcggctgcgg ctgcaatggg cggtgctggc 1380
caaggtggct acggcggcct gggttctcag ggtactagcg gtcgcggcgg tctgggtggc 1440
cagggtgcag gtatggcggc tgcggctgca atgggcggtg ctggccaagg tggctacggc 1500
ggcctgggtt ctcagggtac tagcggtcgc ggcggtctgg gtggccaggg tgcaggtatg 1560
gcggctgcgg ctgcaatggg cggtgctggc caaggtggct acggcggcct gggttctcag 1620
ggtactagcg gtcgcggcgg tctgggtggc cagggtgcag gtatggcggc tgcggctgca 1680
atgggcggtg ctggccaagg tggctacggc ggcctgggtt ctcagggtac tagcggtcgc 1740
ggcggtctgg gtggccaggg tgcaggtatg gcggctgcgg ctgcaatggg cggtgctggc 1800
caaggtggct acggcggcct gggttctcag ggtactagcg gtcgcggcgg tctgggtggc 1860
cagggtgcag gtatggcggc tgcggctgca atgggcggtg ctggccaagg tggctacggc 1920

ggcctgggtt ctcagggtac tagcggtcgc ggcggtctgg gtggccaggg tgcaggtatg 1980

gcggctgcgg ctgcaatggg cggtgctggc caaggtggct acggcggcct gggttctcag 2040

ggtactagcg gtcgcggcgg tctgggtggc cagggtgcag gtatggcggc tgcggctgca 2100

atgggcggtg ctggccaagg tggctacggc ggcctgggtt ctcagggtac tagcggtcgc 2160

ggcggtctgg gtggccaggg tgcaggtatg gcggctgcgg ctgcaatggg cggtgctggc 2220

caaggtggct acggcggcct gggttctcag ggtactagcg gtcgcggcgg tctgggtggc 2280

cagggtgcag gtatggcggc tgcggctgca atgggcggtg ctggccaagg tggctacggc 2340

ggcctgggtt ctcagggtac tagcggtcgc ggcggtctgg gtggccaggg tgcaggtatg 2400

gcggctgcgg ctgcaatggg cggtgctggc caaggtggct acggcggcct gggttctcag 2460

ggtactagcg gtcgcggcgg tctgggtggc cagggtgcag gtatggcggc tgcggctgca 2520

atgggcggtg ctggccaagg tggctacggc ggcctgggtt ctcagggtac tagcggtcgc 2580

ggcggtctgg gtggccaggg tgcaggtatg gcggctgcgg ctgcaatggg cggtgctggc 2640

caaggtggct acggcggcct gggttctcag ggtactagcg gtcgcggcgg tctgggtggc 2700

cagggtgcag gtatggcggc tgcggctgca atgggcggtg ctggccaagg tggctacggc 2760

ggcctgggtt ctcagggtac tagcggtcgc ggcggtctgg gtggccaggg tgcaggtatg 2820

gcggctgcgg ctgcaatggg cggtgctggc caaggtggct acggcggcct gggttctcag 2880

ggtactagcg gtcgcggcgg tctgggtggc cagggtgcag gtatggcggc tgcggctgca 2940

atgggcggtg ctggccaagg tggctacggc ggcctgggtt ctcagggtac tagcggtcgc 3000

ggcggtctgg gtggccaggg tgcaggtatg gcggctgcgg ctgcaatggg cggtgctggc 3060

caaggtggct acggcggcct gggttctcag ggtactagcg gtcgcggcgg tctgggtggc 3120

cagggtgcag gtatggcggc tgcggctgca atgggcggtg ctggccaagg tggctacggc 3180

ggcctgggtt ctcagggtac tagcggtcgc ggcggtctgg gtggccaggg tgcaggtatg 3240

gcggctgcgg ctgcaatggg cggtgctggc caaggtggct acggcggcct gggttctcag 3300

ggtactagcg gtcgcggcgg tctgggtggc cagggtgcag gtatggcggc tgcggctgca 3360

atgggcggtg ctggccaagg tggctacggc ggcctgggtt ctcagggtac tagcggtcgc 3420

ggcggtctgg gtggccaggg tgcaggtatg gcggctgcgg ctgcaatggg cggtgctggc 3480

caaggtggct acggcggcct gggttctcag ggtactagtg gatccgaatt cgagctccgt 3540

cgacaagctt gcggccgcac tcgagcacca ccaccaccac cactgagatc cggctgctaa 3600

<210> 15

<211> 6960
<212> DNA
<213> Artificial

<220>
<223> 64mer silk protein nucleic acid sequence

<400> 15

```
atgcaccatc atcatcatca ttcttctggt ctggtgccac gcggttctgg tatgaaagaa     60
accgctgctg ctaaattcga acgccagcac atggacagcc cagatctggg taccgacgac    120
gacgacaagg ccatggctga tatcggatcc atggctagcg gtcgcggcgg tctgggtggc    180
cagggtgcag gtatggcggc tgcggctgca atgggcggtg ctggccaagg tggctacggc    240
ggcctgggtt ctcagggtac tagcggtcgc ggcggtctgg gtggccaggg tgcaggtatg    300
gcggctgcgg ctgcaatggg cggtgctggc caaggtggct acggcggcct gggttctcag    360
ggtactagcg gtcgcggcgg tctgggtggc cagggtgcag gtatggcggc tgcggctgca    420
atgggcggtg ctggccaagg tggctacggc ggcctgggtt ctcagggtac tagcggtcgc    480
ggcggtctgg gtggccaggg tgcaggtatg gcggctgcgg ctgcaatggg cggtgctggc    540
caaggtggct acggcggcct gggttctcag ggtactagcg gtcgcggcgg tctgggtggc    600
cagggtgcag gtatggcggc tgcggctgca atgggcggtg ctggccaagg tggctacggc    660
ggcctgggtt ctcagggtac tagcggtcgc ggcggtctgg gtggccaggg tgcaggtatg    720
gcggctgcgg ctgcaatggg cggtgctggc caaggtggct acggcggcct gggttctcag    780
ggtactagcg gtcgcggcgg tctgggtggc cagggtgcag gtatggcggc tgcggctgca    840
atgggcggtg ctggccaagg tggctacggc ggcctgggtt ctcagggtac tagcggtcgc    900
ggcggtctgg gtggccaggg tgcaggtatg gcggctgcgg ctgcaatggg cggtgctggc    960
caaggtggct acggcggcct gggttctcag ggtactagcg gtcgcggcgg tctgggtggc   1020
cagggtgcag gtatggcggc tgcggctgca atgggcggtg ctggccaagg tggctacggc   1080
ggcctgggtt ctcagggtac tagcggtcgc ggcggtctgg gtggccaggg tgcaggtatg   1140
gcggctgcgg ctgcaatggg cggtgctggc caaggtggct acggcggcct gggttctcag   1200
ggtactagcg gtcgcggcgg tctgggtggc cagggtgcag gtatggcggc tgcggctgca   1260
atgggcggtg ctggccaagg tggctacggc ggcctgggtt ctcagggtac tagcggtcgc   1320
ggcggtctgg gtggccaggg tgcaggtatg gcggctgcgg ctgcaatggg cggtgctggc   1380
caaggtggct acggcggcct gggttctcag ggtactagcg gtcgcggcgg tctgggtggc   1440
cagggtgcag gtatggcggc tgcggctgca atgggcggtg ctggccaagg tggctacggc   1500
```

ggcctgggtt ctcagggtac tagcggtcgc ggcggtctgg gtggccaggg tgcaggtatg 1560
gcggctgcgg ctgcaatggg cggtgctggc caaggtggct acggcggcct gggttctcag 1620
ggtactagcg gtcgcggcgg tctgggtggc cagggtgcag gtatggcggc tgcggctgca 1680
atgggcggtg ctggccaagg tggctacggc ggcctgggtt ctcagggtac tagcggtcgc 1740
ggcggtctgg gtggccaggg tgcaggtatg gcggctgcgg ctgcaatggg cggtgctggc 1800
caaggtggct acggcggcct gggttctcag ggtactagcg gtcgcggcgg tctgggtggc 1860
cagggtgcag gtatggcggc tgcggctgca atgggcggtg ctggccaagg tggctacggc 1920
ggcctgggtt ctcagggtac tagcggtcgc ggcggtctgg gtggccaggg tgcaggtatg 1980
gcggctgcgg ctgcaatggg cggtgctggc caaggtggct acggcggcct gggttctcag 2040
ggtactagcg gtcgcggcgg tctgggtggc cagggtgcag gtatggcggc tgcggctgca 2100
atgggcggtg ctggccaagg tggctacggc ggcctgggtt ctcagggtac tagcggtcgc 2160
ggcggtctgg gtggccaggg tgcaggtatg gcggctgcgg ctgcaatggg cggtgctggc 2220
caaggtggct acggcggcct gggttctcag ggtactagcg gtcgcggcgg tctgggtggc 2280
cagggtgcag gtatggcggc tgcggctgca atgggcggtg ctggccaagg tggctacggc 2340
ggcctgggtt ctcagggtac tagcggtcgc ggcggtctgg gtggccaggg tgcaggtatg 2400
gcggctgcgg ctgcaatggg cggtgctggc caaggtggct acggcggcct gggttctcag 2460
ggtactagcg gtcgcggcgg tctgggtggc cagggtgcag gtatggcggc tgcggctgca 2520
atgggcggtg ctggccaagg tggctacggc ggcctgggtt ctcagggtac tagcggtcgc 2580
ggcggtctgg gtggccaggg tgcaggtatg gcggctgcgg ctgcaatggg cggtgctggc 2640
caaggtggct acggcggcct gggttctcag ggtactagcg gtcgcggcgg tctgggtggc 2700
cagggtgcag gtatggcggc tgcggctgca atgggcggtg ctggccaagg tggctacggc 2760
ggcctgggtt ctcagggtac tagcggtcgc ggcggtctgg gtggccaggg tgcaggtatg 2820
gcggctgcgg ctgcaatggg cggtgctggc caaggtggct acggcggcct gggttctcag 2880
ggtactagcg gtcgcggcgg tctgggtggc cagggtgcag gtatggcggc tgcggctgca 2940
atgggcggtg ctggccaagg tggctacggc ggcctgggtt ctcagggtac tagcggtcgc 3000
ggcggtctgg gtggccaggg tgcaggtatg gcggctgcgg ctgcaatggg cggtgctggc 3060
caaggtggct acggcggcct gggttctcag ggtactagcg gtcgcggcgg tctgggtggc 3120
cagggtgcag gtatggcggc tgcggctgca atgggcggtg ctggccaagg tggctacggc 3180
ggcctgggtt ctcagggtac tagcggtcgc ggcggtctgg gtggccaggg tgcaggtatg 3240

gcggctgcgg ctgcaatggg cggtgctggc caaggtggct acggcggcct gggttctcag 3300
ggtactagcg gtcgcggcgg tctgggtggc cagggtgcag gtatggcggc tgcggctgca 3360
atgggcggtg ctggccaagg tggctacggc ggcctgggtt ctcagggtac tagcggtcgc 3420
ggcggtctgg gtggccaggg tgcaggtatg gcggctgcgg ctgcaatggg cggtgctggc 3480
caaggtggct acggcggcct gggttctcag ggtactagcg gtcgcggcgg tctgggtggc 3540
cagggtgcag gtatggcggc tgcggctgca atgggcggtg ctggccaagg tggctacggc 3600
ggcctgggtt ctcagggtac tagcggtcgc ggcggtctgg gtggccaggg tgcaggtatg 3660
gcggctgcgg ctgcaatggg cggtgctggc caaggtggct acggcggcct gggttctcag 3720
ggtactagcg gtcgcggcgg tctgggtggc cagggtgcag gtatggcggc tgcggctgca 3780
atgggcggtg ctggccaagg tggctacggc ggcctgggtt ctcagggtac tagcggtcgc 3840
ggcggtctgg gtggccaggg tgcaggtatg gcggctgcgg ctgcaatggg cggtgctggc 3900
caaggtggct acggcggcct gggttctcag ggtactagcg gtcgcggcgg tctgggtggc 3960
cagggtgcag gtatggcggc tgcggctgca atgggcggtg ctggccaagg tggctacggc 4020
ggcctgggtt ctcagggtac tagcggtcgc ggcggtctgg gtggccaggg tgcaggtatg 4080
gcggctgcgg ctgcaatggg cggtgctggc caaggtggct acggcggcct gggttctcag 4140
ggtactagcg gtcgcggcgg tctgggtggc cagggtgcag gtatggcggc tgcggctgca 4200
atgggcggtg ctggccaagg tggctacggc ggcctgggtt ctcagggtac tagcggtcgc 4260
ggcggtctgg gtggccaggg tgcaggtatg gcggctgcgg ctgcaatggg cggtgctggc 4320
caaggtggct acggcggcct gggttctcag ggtactagcg gtcgcggcgg tctgggtggc 4380
cagggtgcag gtatggcggc tgcggctgca atgggcggtg ctggccaagg tggctacggc 4440
ggcctgggtt ctcagggtac tagcggtcgc ggcggtctgg gtggccaggg tgcaggtatg 4500
gcggctgcgg ctgcaatggg cggtgctggc caaggtggct acggcggcct gggttctcag 4560
ggtactagcg gtcgcggcgg tctgggtggc cagggtgcag gtatggcggc tgcggctgca 4620
atgggcggtg ctggccaagg tggctacggc ggcctgggtt ctcagggtac tagcggtcgc 4680
ggcggtctgg gtggccaggg tgcaggtatg gcggctgcgg ctgcaatggg cggtgctggc 4740
caaggtggct acggcggcct gggttctcag ggtactagcg gtcgcggcgg tctgggtggc 4800
cagggtgcag gtatggcggc tgcggctgca atgggcggtg ctggccaagg tggctacggc 4860
ggcctgggtt ctcagggtac tagcggtcgc ggcggtctgg gtggccaggg tgcaggtatg 4920
gcggctgcgg ctgcaatggg cggtgctggc caaggtggct acggcggcct gggttctcag 4980

ggtactagcg gtcgcggcgg tctgggtggc cagggtgcag gtatggcggc tgcggctgca 5040
atgggcggtg ctggccaagg tggctacggc ggcctgggtt ctcagggtac tagcggtcgc 5100
ggcggtctgg gtggccaggg tgcaggtatg gcggctgcgg ctgcaatggg cggtgctggc 5160
caaggtggct acggcggcct gggttctcag ggtactagcg gtcgcggcgg tctgggtggc 5220
cagggtgcag gtatggcggc tgcggctgca atgggcggtg ctggccaagg tggctacggc 5280
ggcctgggtt ctcagggtac tagcggtcgc ggcggtctgg gtggccaggg tgcaggtatg 5340
gcggctgcgg ctgcaatggg cggtgctggc caaggtggct acggcggcct gggttctcag 5400
ggtactagcg gtcgcggcgg tctgggtggc cagggtgcag gtatggcggc tgcggctgca 5460
atgggcggtg ctggccaagg tggctacggc ggcctgggtt ctcagggtac tagcggtcgc 5520
ggcggtctgg gtggccaggg tgcaggtatg gcggctgcgg ctgcaatggg cggtgctggc 5580
caaggtggct acggcggcct gggttctcag ggtactagcg gtcgcggcgg tctgggtggc 5640
cagggtgcag gtatggcggc tgcggctgca atgggcggtg ctggccaagg tggctacggc 5700
ggcctgggtt ctcagggtac tagcggtcgc ggcggtctgg gtggccaggg tgcaggtatg 5760
gcggctgcgg ctgcaatggg cggtgctggc caaggtggct acggcggcct gggttctcag 5820
ggtactagcg gtcgcggcgg tctgggtggc cagggtgcag gtatggcggc tgcggctgca 5880
atgggcggtg ctggccaagg tggctacggc ggcctgggtt ctcagggtac tagcggtcgc 5940
ggcggtctgg gtggccaggg tgcaggtatg gcggctgcgg ctgcaatggg cggtgctggc 6000
caaggtggct acggcggcct gggttctcag ggtactagcg gtcgcggcgg tctgggtggc 6060
cagggtgcag gtatggcggc tgcggctgca atgggcggtg ctggccaagg tggctacggc 6120
ggcctgggtt ctcagggtac tagcggtcgc ggcggtctgg gtggccaggg tgcaggtatg 6180
gcggctgcgg ctgcaatggg cggtgctggc caaggtggct acggcggcct gggttctcag 6240
ggtactagcg gtcgcggcgg tctgggtggc cagggtgcag gtatggcggc tgcggctgca 6300
atgggcggtg ctggccaagg tggctacggc ggcctgggtt ctcagggtac tagcggtcgc 6360
ggcggtctgg gtggccaggg tgcaggtatg gcggctgcgg ctgcaatggg cggtgctggc 6420
caaggtggct acggcggcct gggttctcag ggtactagcg gtcgcggcgg tctgggtggc 6480
cagggtgcag gtatggcggc tgcggctgca atgggcggtg ctggccaagg tggctacggc 6540
ggcctgggtt ctcagggtac tagcggtcgc ggcggtctgg gtggccaggg tgcaggtatg 6600
gcggctgcgg ctgcaatggg cggtgctggc caaggtggct acggcggcct gggttctcag 6660
ggtactagcg gtcgcggcgg tctgggtggc cagggtgcag gtatggcggc tgcggctgca 6720

atgggcggtg ctggccaagg tggctacggc ggcctgggtt ctcagggtac tagcggtcgc 6780

ggcggtctgg gtggccaggg tgcaggtatg gcggctgcgg ctgcaatggg cggtgctggc 6840

caaggtggct acggcggcct gggttctcag ggtactagtg gatccgaatt cgagctccgt 6900

cgacaagctt gcggccgcac tcgagcacca ccaccaccac cactgagatc cggctgctaa 6960

<210> 16
<211> 8640
<212> DNA
<213> Artificial

<220>
<223> 80mer silk protein nucleic acid sequence

<400> 16
atgcaccatc atcatcatca ttcttctggt ctggtgccac gcggttctgg tatgaaagaa 60

accgctgctg ctaaattcga acgccagcac atggacagcc cagatctggg taccgacgac 120

gacgacaagg ccatggctga tatcggatcc atggctagcg gtcgcggcgg tctgggtggc 180

cagggtgcag gtatggcggc tgcggctgca atgggcggtg ctggccaagg tggctacggc 240

ggcctgggtt ctcagggtac tagcggtcgc ggcggtctgg gtggccaggg tgcaggtatg 300

gcggctgcgg ctgcaatggg cggtgctggc caaggtggct acggcggcct gggttctcag 360

ggtactagcg gtcgcggcgg tctgggtggc cagggtgcag gtatggcggc tgcggctgca 420

atgggcggtg ctggccaagg tggctacggc ggcctgggtt ctcagggtac tagcggtcgc 480

ggcggtctgg gtggccaggg tgcaggtatg gcggctgcgg ctgcaatggg cggtgctggc 540

caaggtggct acggcggcct gggttctcag ggtactagcg gtcgcggcgg tctgggtggc 600

cagggtgcag gtatggcggc tgcggctgca atgggcggtg ctggccaagg tggctacggc 660

ggcctgggtt ctcagggtac tagcggtcgc ggcggtctgg gtggccaggg tgcaggtatg 720

gcggctgcgg ctgcaatggg cggtgctggc caaggtggct acggcggcct gggttctcag 780

ggtactagcg gtcgcggcgg tctgggtggc cagggtgcag gtatggcggc tgcggctgca 840

atgggcggtg ctggccaagg tggctacggc ggcctgggtt ctcagggtac tagcggtcgc 900

ggcggtctgg gtggccaggg tgcaggtatg gcggctgcgg ctgcaatggg cggtgctggc 960

caaggtggct acggcggcct gggttctcag ggtactagcg gtcgcggcgg tctgggtggc 1020

cagggtgcag gtatggcggc tgcggctgca atgggcggtg ctggccaagg tggctacggc 1080

ggcctgggtt ctcagggtac tagcggtcgc ggcggtctgg gtggccaggg tgcaggtatg 1140

gcggctgcgg ctgcaatggg cggtgctggc caaggtggct acggcggcct gggttctcag 1200

ggtactagcg gtcgcggcgg tctgggtggc cagggtgcag gtatggcggc tgcggctgca 1260
atgggcggtg ctggccaagg tggctacggc ggcctgggtt ctcagggtac tagcggtcgc 1320
ggcggtctgg gtggccaggg tgcaggtatg gcggctgcgg ctgcaatggg cggtgctggc 1380
caaggtggct acggcggcct gggttctcag ggtactagcg gtcgcggcgg tctgggtggc 1440
cagggtgcag gtatggcggc tgcggctgca atgggcggtg ctggccaagg tggctacggc 1500
ggcctgggtt ctcagggtac tagcggtcgc ggcggtctgg gtggccaggg tgcaggtatg 1560
gcggctgcgg ctgcaatggg cggtgctggc caaggtggct acggcggcct gggttctcag 1620
ggtactagcg gtcgcggcgg tctgggtggc cagggtgcag gtatggcggc tgcggctgca 1680
atgggcggtg ctggccaagg tggctacggc ggcctgggtt ctcagggtac tagcggtcgc 1740
ggcggtctgg gtggccaggg tgcaggtatg gcggctgcgg ctgcaatggg cggtgctggc 1800
caaggtggct acggcggcct gggttctcag ggtactagcg gtcgcggcgg tctgggtggc 1860
cagggtgcag gtatggcggc tgcggctgca atgggcggtg ctggccaagg tggctacggc 1920
ggcctgggtt ctcagggtac tagcggtcgc ggcggtctgg gtggccaggg tgcaggtatg 1980
gcggctgcgg ctgcaatggg cggtgctggc caaggtggct acggcggcct gggttctcag 2040
ggtactagcg gtcgcggcgg tctgggtggc cagggtgcag gtatggcggc tgcggctgca 2100
atgggcggtg ctggccaagg tggctacggc ggcctgggtt ctcagggtac tagcggtcgc 2160
ggcggtctgg gtggccaggg tgcaggtatg gcggctgcgg ctgcaatggg cggtgctggc 2220
caaggtggct acggcggcct gggttctcag ggtactagcg gtcgcggcgg tctgggtggc 2280
cagggtgcag gtatggcggc tgcggctgca atgggcggtg ctggccaagg tggctacggc 2340
ggcctgggtt ctcagggtac tagcggtcgc ggcggtctgg gtggccaggg tgcaggtatg 2400
gcggctgcgg ctgcaatggg cggtgctggc caaggtggct acggcggcct gggttctcag 2460
ggtactagcg gtcgcggcgg tctgggtggc cagggtgcag gtatggcggc tgcggctgca 2520
atgggcggtg ctggccaagg tggctacggc ggcctgggtt ctcagggtac tagcggtcgc 2580
ggcggtctgg gtggccaggg tgcaggtatg gcggctgcgg ctgcaatggg cggtgctggc 2640
caaggtggct acggcggcct gggttctcag ggtactagcg gtcgcggcgg tctgggtggc 2700
cagggtgcag gtatggcggc tgcggctgca atgggcggtg ctggccaagg tggctacggc 2760
ggcctgggtt ctcagggtac tagcggtcgc ggcggtctgg gtggccaggg tgcaggtatg 2820
gcggctgcgg ctgcaatggg cggtgctggc caaggtggct acggcggcct gggttctcag 2880
ggtactagcg gtcgcggcgg tctgggtggc cagggtgcag gtatggcggc tgcggctgca 2940

atgggcggtg ctggccaagg tggctacggc ggcctgggtt ctcagggtac tagcggtcgc 3000
ggcggtctgg gtggccaggg tgcaggtatg gcggctgcgg ctgcaatggg cggtgctggc 3060
caaggtggct acggcggcct gggttctcag ggtactagcg gtcgcggcgg tctgggtggc 3120
cagggtgcag gtatggcggc tgcggctgca atgggcggtg ctggccaagg tggctacggc 3180
ggcctgggtt ctcagggtac tagcggtcgc ggcggtctgg gtggccaggg tgcaggtatg 3240
gcggctgcgg ctgcaatggg cggtgctggc caaggtggct acggcggcct gggttctcag 3300
ggtactagcg gtcgcggcgg tctgggtggc cagggtgcag gtatggcggc tgcggctgca 3360
atgggcggtg ctggccaagg tggctacggc ggcctgggtt ctcagggtac tagcggtcgc 3420
ggcggtctgg gtggccaggg tgcaggtatg gcggctgcgg ctgcaatggg cggtgctggc 3480
caaggtggct acggcggcct gggttctcag ggtactagcg gtcgcggcgg tctgggtggc 3540
cagggtgcag gtatggcggc tgcggctgca atgggcggtg ctggccaagg tggctacggc 3600
ggcctgggtt ctcagggtac tagcggtcgc ggcggtctgg gtggccaggg tgcaggtatg 3660
gcggctgcgg ctgcaatggg cggtgctggc caaggtggct acggcggcct gggttctcag 3720
ggtactagcg gtcgcggcgg tctgggtggc cagggtgcag gtatggcggc tgcggctgca 3780
atgggcggtg ctggccaagg tggctacggc ggcctgggtt ctcagggtac tagcggtcgc 3840
ggcggtctgg gtggccaggg tgcaggtatg gcggctgcgg ctgcaatggg cggtgctggc 3900
caaggtggct acggcggcct gggttctcag ggtactagcg gtcgcggcgg tctgggtggc 3960
cagggtgcag gtatggcggc tgcggctgca atgggcggtg ctggccaagg tggctacggc 4020
ggcctgggtt ctcagggtac tagcggtcgc ggcggtctgg gtggccaggg tgcaggtatg 4080
gcggctgcgg ctgcaatggg cggtgctggc caaggtggct acggcggcct gggttctcag 4140
ggtactagcg gtcgcggcgg tctgggtggc cagggtgcag gtatggcggc tgcggctgca 4200
atgggcggtg ctggccaagg tggctacggc ggcctgggtt ctcagggtac tagcggtcgc 4260
ggcggtctgg gtggccaggg tgcaggtatg gcggctgcgg ctgcaatggg cggtgctggc 4320
caaggtggct acggcggcct gggttctcag ggtactagcg gtcgcggcgg tctgggtggc 4380
cagggtgcag gtatggcggc tgcggctgca atgggcggtg ctggccaagg tggctacggc 4440
ggcctgggtt ctcagggtac tagcggtcgc ggcggtctgg gtggccaggg tgcaggtatg 4500
gcggctgcgg ctgcaatggg cggtgctggc caaggtggct acggcggcct gggttctcag 4560
ggtactagcg gtcgcggcgg tctgggtggc cagggtgcag gtatggcggc tgcggctgca 4620
atgggcggtg ctggccaagg tggctacggc ggcctgggtt ctcagggtac tagcggtcgc 4680

ggcggtctgg gtggccaggg tgcaggtatg gcggctgcgg ctgcaatggg cggtgctggc 4740
caaggtggct acggcggcct gggttctcag ggtactagcg gtcgcggcgg tctgggtggc 4800
cagggtgcag gtatggcggc tgcggctgca atgggcggtg ctggccaagg tggctacggc 4860
ggcctgggtt ctcagggtac tagcggtcgc ggcggtctgg gtggccaggg tgcaggtatg 4920
gcggctgcgg ctgcaatggg cggtgctggc caaggtggct acggcggcct gggttctcag 4980
ggtactagcg gtcgcggcgg tctgggtggc cagggtgcag gtatggcggc tgcggctgca 5040
atgggcggtg ctggccaagg tggctacggc ggcctgggtt ctcagggtac tagcggtcgc 5100
ggcggtctgg gtggccaggg tgcaggtatg gcggctgcgg ctgcaatggg cggtgctggc 5160
caaggtggct acggcggcct gggttctcag ggtactagcg gtcgcggcgg tctgggtggc 5220
cagggtgcag gtatggcggc tgcggctgca atgggcggtg ctggccaagg tggctacggc 5280
ggcctgggtt ctcagggtac tagcggtcgc ggcggtctgg gtggccaggg tgcaggtatg 5340
gcggctgcgg ctgcaatggg cggtgctggc caaggtggct acggcggcct gggttctcag 5400
ggtactagcg gtcgcggcgg tctgggtggc cagggtgcag gtatggcggc tgcggctgca 5460
atgggcggtg ctggccaagg tggctacggc ggcctgggtt ctcagggtac tagcggtcgc 5520
ggcggtctgg gtggccaggg tgcaggtatg gcggctgcgg ctgcaatggg cggtgctggc 5580
caaggtggct acggcggcct gggttctcag ggtactagcg gtcgcggcgg tctgggtggc 5640
cagggtgcag gtatggcggc tgcggctgca atgggcggtg ctggccaagg tggctacggc 5700
ggcctgggtt ctcagggtac tagcggtcgc ggcggtctgg gtggccaggg tgcaggtatg 5760
gcggctgcgg ctgcaatggg cggtgctggc caaggtggct acggcggcct gggttctcag 5820
ggtactagcg gtcgcggcgg tctgggtggc cagggtgcag gtatggcggc tgcggctgca 5880
atgggcggtg ctggccaagg tggctacggc ggcctgggtt ctcagggtac tagcggtcgc 5940
ggcggtctgg gtggccaggg tgcaggtatg gcggctgcgg ctgcaatggg cggtgctggc 6000
caaggtggct acggcggcct gggttctcag ggtactagcg gtcgcggcgg tctgggtggc 6060
cagggtgcag gtatggcggc tgcggctgca atgggcggtg ctggccaagg tggctacggc 6120
ggcctgggtt ctcagggtac tagcggtcgc ggcggtctgg gtggccaggg tgcaggtatg 6180
gcggctgcgg ctgcaatggg cggtgctggc caaggtggct acggcggcct gggttctcag 6240
ggtactagcg gtcgcggcgg tctgggtggc cagggtgcag gtatggcggc tgcggctgca 6300
atgggcggtg ctggccaagg tggctacggc ggcctgggtt ctcagggtac tagcggtcgc 6360
ggcggtctgg gtggccaggg tgcaggtatg gcggctgcgg ctgcaatggg cggtgctggc 6420

caaggtggct acggcggcct gggttctcag ggtactagcg gtcgcggcgg tctgggtggc 6480
cagggtgcag gtatggcggc tgcggctgca atgggcggtg ctggccaagg tggctacggc 6540
ggcctgggtt ctcagggtac tagcggtcgc ggcggtctgg gtggccaggg tgcaggtatg 6600
gcggctgcgg ctgcaatggg cggtgctggc caaggtggct acggcggcct gggttctcag 6660
ggtactagcg gtcgcggcgg tctgggtggc cagggtgcag gtatggcggc tgcggctgca 6720
atgggcggtg ctggccaagg tggctacggc ggcctgggtt ctcagggtac tagcggtcgc 6780
ggcggtctgg gtggccaggg tgcaggtatg gcggctgcgg ctgcaatggg cggtgctggc 6840
caaggtggct acggcggcct gggttctcag ggtactagcg gtcgcggcgg tctgggtggc 6900
cagggtgcag gtatggcggc tgcggctgca atgggcggtg ctggccaagg tggctacggc 6960
ggcctgggtt ctcagggtac tagcggtcgc ggcggtctgg gtggccaggg tgcaggtatg 7020
gcggctgcgg ctgcaatggg cggtgctggc caaggtggct acggcggcct gggttctcag 7080
ggtactagcg gtcgcggcgg tctgggtggc cagggtgcag gtatggcggc tgcggctgca 7140
atgggcggtg ctggccaagg tggctacggc ggcctgggtt ctcagggtac tagcggtcgc 7200
ggcggtctgg gtggccaggg tgcaggtatg gcggctgcgg ctgcaatggg cggtgctggc 7260
caaggtggct acggcggcct gggttctcag ggtactagcg gtcgcggcgg tctgggtggc 7320
cagggtgcag gtatggcggc tgcggctgca atgggcggtg ctggccaagg tggctacggc 7380
ggcctgggtt ctcagggtac tagcggtcgc ggcggtctgg gtggccaggg tgcaggtatg 7440
gcggctgcgg ctgcaatggg cggtgctggc caaggtggct acggcggcct gggttctcag 7500
ggtactagcg gtcgcggcgg tctgggtggc cagggtgcag gtatggcggc tgcggctgca 7560
atgggcggtg ctggccaagg tggctacggc ggcctgggtt ctcagggtac tagcggtcgc 7620
ggcggtctgg gtggccaggg tgcaggtatg gcggctgcgg ctgcaatggg cggtgctggc 7680
caaggtggct acggcggcct gggttctcag ggtactagcg gtcgcggcgg tctgggtggc 7740
cagggtgcag gtatggcggc tgcggctgca atgggcggtg ctggccaagg tggctacggc 7800
ggcctgggtt ctcagggtac tagcggtcgc ggcggtctgg gtggccaggg tgcaggtatg 7860
gcggctgcgg ctgcaatggg cggtgctggc caaggtggct acggcggcct gggttctcag 7920
ggtactagcg gtcgcggcgg tctgggtggc cagggtgcag gtatggcggc tgcggctgca 7980
atgggcggtg ctggccaagg tggctacggc ggcctgggtt ctcagggtac tagcggtcgc 8040
ggcggtctgg gtggccaggg tgcaggtatg gcggctgcgg ctgcaatggg cggtgctggc 8100
caaggtggct acggcggcct gggttctcag ggtactagcg gtcgcggcgg tctgggtggc 8160

caggggtgcag gtatggcggc tgcggctgca atgggcggtg ctggccaagg tggctacggc 8220

ggcctgggtt ctcagggtac tagcggtcgc ggcggtctgg gtggccaggg tgcaggtatg 8280

gcggctgcgg ctgcaatggg cggtgctggc caaggtggct acggcggcct gggttctcag 8340

ggtactagcg gtcgcggcgg tctgggtggc cagggtgcag gtatggcggc tgcggctgca 8400

atgggcggtg ctggccaagg tggctacggc ggcctgggtt ctcagggtac tagcggtcgc 8460

ggcggtctgg gtggccaggg tgcaggtatg gcggctgcgg ctgcaatggg cggtgctggc 8520

caaggtggct acggcggcct gggttctcag ggtactagtg gatccgaatt cgagctccgt 8580

cgacaagctt gcggccgcac tcgagcacca ccaccaccac cactgagatc cggctgctaa 8640

<210> 17
<211> 10320
<212> DNA
<213> Artificial

<220>
<223> 96mer silk protein nucleic acid sequence

<400> 17
atgcaccatc atcatcatca ttcttctggt ctggtgccac gcggttctgg tatgaaagaa 60

accgctgctg ctaaattcga acgccagcac atggacagcc cagatctggg taccgacgac 120

gacgacaagg ccatggctga tatcggatcc atggctagcg gtcgcggcgg tctgggtggc 180

cagggtgcag gtatggcggc tgcggctgca atgggcggtg ctggccaagg tggctacggc 240

ggcctgggtt ctcagggtac tagcggtcgc ggcggtctgg gtggccaggg tgcaggtatg 300

gcggctgcgg ctgcaatggg cggtgctggc caaggtggct acggcggcct gggttctcag 360

ggtactagcg gtcgcggcgg tctgggtggc cagggtgcag gtatggcggc tgcggctgca 420

atgggcggtg ctggccaagg tggctacggc ggcctgggtt ctcagggtac tagcggtcgc 480

ggcggtctgg gtggccaggg tgcaggtatg gcggctgcgg ctgcaatggg cggtgctggc 540

caaggtggct acggcggcct gggttctcag ggtactagcg gtcgcggcgg tctgggtggc 600

cagggtgcag gtatggcggc tgcggctgca atgggcggtg ctggccaagg tggctacggc 660

ggcctgggtt ctcagggtac tagcggtcgc ggcggtctgg gtggccaggg tgcaggtatg 720

gcggctgcgg ctgcaatggg cggtgctggc caaggtggct acggcggcct gggttctcag 780

ggtactagcg gtcgcggcgg tctgggtggc cagggtgcag gtatggcggc tgcggctgca 840

atgggcggtg ctggccaagg tggctacggc ggcctgggtt ctcagggtac tagcggtcgc 900

ggcggtctgg gtggccaggg tgcaggtatg gcggctgcgg ctgcaatggg cggtgctggc 960

caaggtggct acggcggcct gggttctcag ggtactagcg gtcgcggcgg tctgggtggc 1020
cagggtgcag gtatggcggc tgcggctgca atgggcggtg ctggccaagg tggctacggc 1080
ggcctgggtt ctcagggtac tagcggtcgc ggcggtctgg gtggccaggg tgcaggtatg 1140
gcggctgcgg ctgcaatggg cggtgctggc caaggtggct acggcggcct gggttctcag 1200
ggtactagcg gtcgcggcgg tctgggtggc cagggtgcag gtatggcggc tgcggctgca 1260
atgggcggtg ctggccaagg tggctacggc ggcctgggtt ctcagggtac tagcggtcgc 1320
ggcggtctgg gtggccaggg tgcaggtatg gcggctgcgg ctgcaatggg cggtgctggc 1380
caaggtggct acggcggcct gggttctcag ggtactagcg gtcgcggcgg tctgggtggc 1440
cagggtgcag gtatggcggc tgcggctgca atgggcggtg ctggccaagg tggctacggc 1500
ggcctgggtt ctcagggtac tagcggtcgc ggcggtctgg gtggccaggg tgcaggtatg 1560
gcggctgcgg ctgcaatggg cggtgctggc caaggtggct acggcggcct gggttctcag 1620
ggtactagcg gtcgcggcgg tctgggtggc cagggtgcag gtatggcggc tgcggctgca 1680
atgggcggtg ctggccaagg tggctacggc ggcctgggtt ctcagggtac tagcggtcgc 1740
ggcggtctgg gtggccaggg tgcaggtatg gcggctgcgg ctgcaatggg cggtgctggc 1800
caaggtggct acggcggcct gggttctcag ggtactagcg gtcgcggcgg tctgggtggc 1860
cagggtgcag gtatggcggc tgcggctgca atgggcggtg ctggccaagg tggctacggc 1920
ggcctgggtt ctcagggtac tagcggtcgc ggcggtctgg gtggccaggg tgcaggtatg 1980
gcggctgcgg ctgcaatggg cggtgctggc caaggtggct acggcggcct gggttctcag 2040
ggtactagcg gtcgcggcgg tctgggtggc cagggtgcag gtatggcggc tgcggctgca 2100
atgggcggtg ctggccaagg tggctacggc ggcctgggtt ctcagggtac tagcggtcgc 2160
ggcggtctgg gtggccaggg tgcaggtatg gcggctgcgg ctgcaatggg cggtgctggc 2220
caaggtggct acggcggcct gggttctcag ggtactagcg gtcgcggcgg tctgggtggc 2280
cagggtgcag gtatggcggc tgcggctgca atgggcggtg ctggccaagg tggctacggc 2340
ggcctgggtt ctcagggtac tagcggtcgc ggcggtctgg gtggccaggg tgcaggtatg 2400
gcggctgcgg ctgcaatggg cggtgctggc caaggtggct acggcggcct gggttctcag 2460
ggtactagcg gtcgcggcgg tctgggtggc cagggtgcag gtatggcggc tgcggctgca 2520
atgggcggtg ctggccaagg tggctacggc ggcctgggtt ctcagggtac tagcggtcgc 2580
ggcggtctgg gtggccaggg tgcaggtatg gcggctgcgg ctgcaatggg cggtgctggc 2640
caaggtggct acggcggcct gggttctcag ggtactagcg gtcgcggcgg tctgggtggc 2700

cagggtgcag gtatggcggc tgcggctgca atgggcggtg ctggccaagg tggctacggc 2760
ggcctgggtt ctcagggtac tagcggtcgc ggcggtctgg gtggccaggg tgcaggtatg 2820
gcggctgcgg ctgcaatggg cggtgctggc caaggtggct acggcggcct gggttctcag 2880
ggtactagcg gtcgcggcgg tctgggtggc cagggtgcag gtatggcggc tgcggctgca 2940
atgggcggtg ctggccaagg tggctacggc ggcctgggtt ctcagggtac tagcggtcgc 3000
ggcggtctgg gtggccaggg tgcaggtatg gcggctgcgg ctgcaatggg cggtgctggc 3060
caaggtggct acggcggcct gggttctcag ggtactagcg gtcgcggcgg tctgggtggc 3120
cagggtgcag gtatggcggc tgcggctgca atgggcggtg ctggccaagg tggctacggc 3180
ggcctgggtt ctcagggtac tagcggtcgc ggcggtctgg gtggccaggg tgcaggtatg 3240
gcggctgcgg ctgcaatggg cggtgctggc caaggtggct acggcggcct gggttctcag 3300
ggtactagcg gtcgcggcgg tctgggtggc cagggtgcag gtatggcggc tgcggctgca 3360
atgggcggtg ctggccaagg tggctacggc ggcctgggtt ctcagggtac tagcggtcgc 3420
ggcggtctgg gtggccaggg tgcaggtatg gcggctgcgg ctgcaatggg cggtgctggc 3480
caaggtggct acggcggcct gggttctcag ggtactagcg gtcgcggcgg tctgggtggc 3540
cagggtgcag gtatggcggc tgcggctgca atgggcggtg ctggccaagg tggctacggc 3600
ggcctgggtt ctcagggtac tagcggtcgc ggcggtctgg gtggccaggg tgcaggtatg 3660
gcggctgcgg ctgcaatggg cggtgctggc caaggtggct acggcggcct gggttctcag 3720
ggtactagcg gtcgcggcgg tctgggtggc cagggtgcag gtatggcggc tgcggctgca 3780
atgggcggtg ctggccaagg tggctacggc ggcctgggtt ctcagggtac tagcggtcgc 3840
ggcggtctgg gtggccaggg tgcaggtatg gcggctgcgg ctgcaatggg cggtgctggc 3900
caaggtggct acggcggcct gggttctcag ggtactagcg gtcgcggcgg tctgggtggc 3960
cagggtgcag gtatggcggc tgcggctgca atgggcggtg ctggccaagg tggctacggc 4020
ggcctgggtt ctcagggtac tagcggtcgc ggcggtctgg gtggccaggg tgcaggtatg 4080
gcggctgcgg ctgcaatggg cggtgctggc caaggtggct acggcggcct gggttctcag 4140
ggtactagcg gtcgcggcgg tctgggtggc cagggtgcag gtatggcggc tgcggctgca 4200
atgggcggtg ctggccaagg tggctacggc ggcctgggtt ctcagggtac tagcggtcgc 4260
ggcggtctgg gtggccaggg tgcaggtatg gcggctgcgg ctgcaatggg cggtgctggc 4320
caaggtggct acggcggcct gggttctcag ggtactagcg gtcgcggcgg tctgggtggc 4380
cagggtgcag gtatggcggc tgcggctgca atgggcggtg ctggccaagg tggctacggc 4440

ggcctgggtt ctcagggtac tagcggtcgc ggcggtctgg gtggccaggg tgcaggtatg 4500

gcggctgcgg ctgcaatggg cggtgctggc caaggtggct acggcggcct gggttctcag 4560

ggtactagcg gtcgcggcgg tctgggtggc cagggtgcag gtatggcggc tgcggctgca 4620

atgggcggtg ctggccaagg tggctacggc ggcctgggtt ctcagggtac tagcggtcgc 4680

ggcggtctgg gtggccaggg tgcaggtatg gcggctgcgg ctgcaatggg cggtgctggc 4740

caaggtggct acggcggcct gggttctcag ggtactagcg gtcgcggcgg tctgggtggc 4800

cagggtgcag gtatggcggc tgcggctgca atgggcggtg ctggccaagg tggctacggc 4860

ggcctgggtt ctcagggtac tagcggtcgc ggcggtctgg gtggccaggg tgcaggtatg 4920

gcggctgcgg ctgcaatggg cggtgctggc caaggtggct acggcggcct gggttctcag 4980

ggtactagcg gtcgcggcgg tctgggtggc cagggtgcag gtatggcggc tgcggctgca 5040

atgggcggtg ctggccaagg tggctacggc ggcctgggtt ctcagggtac tagcggtcgc 5100

ggcggtctgg gtggccaggg tgcaggtatg gcggctgcgg ctgcaatggg cggtgctggc 5160

caaggtggct acggcggcct gggttctcag ggtactagcg gtcgcggcgg tctgggtggc 5220

cagggtgcag gtatggcggc tgcggctgca atgggcggtg ctggccaagg tggctacggc 5280

ggcctgggtt ctcagggtac tagcggtcgc ggcggtctgg gtggccaggg tgcaggtatg 5340

gcggctgcgg ctgcaatggg cggtgctggc caaggtggct acggcggcct gggttctcag 5400

ggtactagcg gtcgcggcgg tctgggtggc cagggtgcag gtatggcggc tgcggctgca 5460

atgggcggtg ctggccaagg tggctacggc ggcctgggtt ctcagggtac tagcggtcgc 5520

ggcggtctgg gtggccaggg tgcaggtatg gcggctgcgg ctgcaatggg cggtgctggc 5580

caaggtggct acggcggcct gggttctcag ggtactagcg gtcgcggcgg tctgggtggc 5640

cagggtgcag gtatggcggc tgcggctgca atgggcggtg ctggccaagg tggctacggc 5700

ggcctgggtt ctcagggtac tagcggtcgc ggcggtctgg gtggccaggg tgcaggtatg 5760

gcggctgcgg ctgcaatggg cggtgctggc caaggtggct acggcggcct gggttctcag 5820

ggtactagcg gtcgcggcgg tctgggtggc cagggtgcag gtatggcggc tgcggctgca 5880

atgggcggtg ctggccaagg tggctacggc ggcctgggtt ctcagggtac tagcggtcgc 5940

ggcggtctgg gtggccaggg tgcaggtatg gcggctgcgg ctgcaatggg cggtgctggc 6000

caaggtggct acggcggcct gggttctcag ggtactagcg gtcgcggcgg tctgggtggc 6060

cagggtgcag gtatggcggc tgcggctgca atgggcggtg ctggccaagg tggctacggc 6120

ggcctgggtt ctcagggtac tagcggtcgc ggcggtctgg gtggccaggg tgcaggtatg 6180

gcggctgcgg ctgcaatggg cggtgctggc caaggtggct acggcggcct gggttctcag 6240
ggtactagcg gtcgcggcgg tctgggtggc cagggtgcag gtatggcggc tgcggctgca 6300
atgggcggtg ctggccaagg tggctacggc ggcctgggtt ctcagggtac tagcggtcgc 6360
ggcggtctgg gtggccaggg tgcaggtatg gcggctgcgg ctgcaatggg cggtgctggc 6420
caaggtggct acggcggcct gggttctcag ggtactagcg gtcgcggcgg tctgggtggc 6480
cagggtgcag gtatggcggc tgcggctgca atgggcggtg ctggccaagg tggctacggc 6540
ggcctgggtt ctcagggtac tagcggtcgc ggcggtctgg gtggccaggg tgcaggtatg 6600
gcggctgcgg ctgcaatggg cggtgctggc caaggtggct acggcggcct gggttctcag 6660
ggtactagcg gtcgcggcgg tctgggtggc cagggtgcag gtatggcggc tgcggctgca 6720
atgggcggtg ctggccaagg tggctacggc ggcctgggtt ctcagggtac tagcggtcgc 6780
ggcggtctgg gtggccaggg tgcaggtatg gcggctgcgg ctgcaatggg cggtgctggc 6840
caaggtggct acggcggcct gggttctcag ggtactagcg gtcgcggcgg tctgggtggc 6900
cagggtgcag gtatggcggc tgcggctgca atgggcggtg ctggccaagg tggctacggc 6960
ggcctgggtt ctcagggtac tagcggtcgc ggcggtctgg gtggccaggg tgcaggtatg 7020
gcggctgcgg ctgcaatggg cggtgctggc caaggtggct acggcggcct gggttctcag 7080
ggtactagcg gtcgcggcgg tctgggtggc cagggtgcag gtatggcggc tgcggctgca 7140
atgggcggtg ctggccaagg tggctacggc ggcctgggtt ctcagggtac tagcggtcgc 7200
ggcggtctgg gtggccaggg tgcaggtatg gcggctgcgg ctgcaatggg cggtgctggc 7260
caaggtggct acggcggcct gggttctcag ggtactagcg gtcgcggcgg tctgggtggc 7320
cagggtgcag gtatggcggc tgcggctgca atgggcggtg ctggccaagg tggctacggc 7380
ggcctgggtt ctcagggtac tagcggtcgc ggcggtctgg gtggccaggg tgcaggtatg 7440
gcggctgcgg ctgcaatggg cggtgctggc caaggtggct acggcggcct gggttctcag 7500
ggtactagcg gtcgcggcgg tctgggtggc cagggtgcag gtatggcggc tgcggctgca 7560
atgggcggtg ctggccaagg tggctacggc ggcctgggtt ctcagggtac tagcggtcgc 7620
ggcggtctgg gtggccaggg tgcaggtatg gcggctgcgg ctgcaatggg cggtgctggc 7680
caaggtggct acggcggcct gggttctcag ggtactagcg gtcgcggcgg tctgggtggc 7740
cagggtgcag gtatggcggc tgcggctgca atgggcggtg ctggccaagg tggctacggc 7800
ggcctgggtt ctcagggtac tagcggtcgc ggcggtctgg gtggccaggg tgcaggtatg 7860
gcggctgcgg ctgcaatggg cggtgctggc caaggtggct acggcggcct gggttctcag 7920

ggtactagcg gtcgcggcgg tctgggtggc cagggtgcag gtatggcggc tgcggctgca 7980
atgggcggtg ctggccaagg tggctacggc ggcctgggtt ctcagggtac tagcggtcgc 8040
ggcggtctgg gtggccaggg tgcaggtatg gcggctgcgg ctgcaatggg cggtgctggc 8100
caaggtggct acggcggcct gggttctcag ggtactagcg gtcgcggcgg tctgggtggc 8160
cagggtgcag gtatggcggc tgcggctgca atgggcggtg ctggccaagg tggctacggc 8220
ggcctgggtt ctcagggtac tagcggtcgc ggcggtctgg gtggccaggg tgcaggtatg 8280
gcggctgcgg ctgcaatggg cggtgctggc caaggtggct acggcggcct gggttctcag 8340
ggtactagcg gtcgcggcgg tctgggtggc cagggtgcag gtatggcggc tgcggctgca 8400
atgggcggtg ctggccaagg tggctacggc ggcctgggtt ctcagggtac tagcggtcgc 8460
ggcggtctgg gtggccaggg tgcaggtatg gcggctgcgg ctgcaatggg cggtgctggc 8520
caaggtggct acggcggcct gggttctcag ggtactagcg gtcgcggcgg tctgggtggc 8580
cagggtgcag gtatggcggc tgcggctgca atgggcggtg ctggccaagg tggctacggc 8640
ggcctgggtt ctcagggtac tagcggtcgc ggcggtctgg gtggccaggg tgcaggtatg 8700
gcggctgcgg ctgcaatggg cggtgctggc caaggtggct acggcggcct gggttctcag 8760
ggtactagcg gtcgcggcgg tctgggtggc cagggtgcag gtatggcggc tgcggctgca 8820
atgggcggtg ctggccaagg tggctacggc ggcctgggtt ctcagggtac tagcggtcgc 8880
ggcggtctgg gtggccaggg tgcaggtatg gcggctgcgg ctgcaatggg cggtgctggc 8940
caaggtggct acggcggcct gggttctcag ggtactagcg gtcgcggcgg tctgggtggc 9000
cagggtgcag gtatggcggc tgcggctgca atgggcggtg ctggccaagg tggctacggc 9060
ggcctgggtt ctcagggtac tagcggtcgc ggcggtctgg gtggccaggg tgcaggtatg 9120
gcggctgcgg ctgcaatggg cggtgctggc caaggtggct acggcggcct gggttctcag 9180
ggtactagcg gtcgcggcgg tctgggtggc cagggtgcag gtatggcggc tgcggctgca 9240
atgggcggtg ctggccaagg tggctacggc ggcctgggtt ctcagggtac tagcggtcgc 9300
ggcggtctgg gtggccaggg tgcaggtatg gcggctgcgg ctgcaatggg cggtgctggc 9360
caaggtggct acggcggcct gggttctcag ggtactagcg gtcgcggcgg tctgggtggc 9420
cagggtgcag gtatggcggc tgcggctgca atgggcggtg ctggccaagg tggctacggc 9480
ggcctgggtt ctcagggtac tagcggtcgc ggcggtctgg gtggccaggg tgcaggtatg 9540
gcggctgcgg ctgcaatggg cggtgctggc caaggtggct acggcggcct gggttctcag 9600
ggtactagcg gtcgcggcgg tctgggtggc cagggtgcag gtatggcggc tgcggctgca 9660

atgggcggtg ctggccaagg tggctacggc ggcctgggtt ctcagggtac tagcggtcgc 9720

ggcggtctgg gtggccaggg tgcaggtatg gcggctgcgg ctgcaatggg cggtgctggc 9780

caaggtggct acggcggcct gggttctcag ggtactagcg gtcgcggcgg tctgggtggc 9840

cagggtgcag gtatggcggc tgcggctgca atgggcggtg ctggccaagg tggctacggc 9900

ggcctgggtt ctcagggtac tagcggtcgc ggcggtctgg gtggccaggg tgcaggtatg 9960

gcggctgcgg ctgcaatggg cggtgctggc caaggtggct acggcggcct gggttctcag 10020

ggtactagcg gtcgcggcgg tctgggtggc cagggtgcag gtatggcggc tgcggctgca 10080

atgggcggtg ctggccaagg tggctacggc ggcctgggtt ctcagggtac tagcggtcgc 10140

ggcggtctgg gtggccaggg tgcaggtatg gcggctgcgg ctgcaatggg cggtgctggc 10200

caaggtggct acggcggcct gggttctcag ggtactagtg gatccgaatt cgagctccgt 10260

cgacaagctt gcggccgcac tcgagcacca ccaccaccac cactgagatc cggctgctaa 10320

<210> 18
<211> 105
<212> DNA
<213> Artificial

<220>
<223> nucleic acid sequence encoding amino acid sequence of SEQ Number 1

<400> 18
agcggtcgcg gcggtctggg tggccagggt gcaggtatgg cggctgcggc tgcaatgggc 60

ggtgctggcc aaggtggcta cggcggcctg ggttctcagg gtact 105

<210> 19
<211> 31
<212> DNA
<213> Artificial

<220>
<223> PCR primer

<400> 19
gctcgatatc taacgacgca gaaatgcgaa a 31

<210> 20
<211> 33
<212> DNA
<213> Artificial

<220>
<223> PCR primer

<400> 20
cattggatcc taagattaca gcctgaggct gtg 33

<210> 21
<211> 35
<212> DNA
<213> Artificial

<220>
<223> PCR primer

<400> 21
ggctcgcatg ctcatgtttg acagcttatc atcga 35

<210> 22
<211> 40
<212> DNA
<213> Artificial

<220>
<223> PCR primer

<400> 22
attgtcgact gctgcagtaa gattacagcc tgaggctgtg 40

## Description

### TECHNICAL FIELD

The present invention relates to a high-molecular-weight recombinant silk or silk-like protein having a molecular weight which is substantially similar to that of native silk protein, and to a micro- or nano-sized spider silk or silk-like fiber having improved physical properties, produced therefrom.

### BACKGROUND ART

Spider dragline silk that is used by spiders as the safety line and the web frame is very strong and elastic. Indeed, spider dragline silk is five times stronger by weight than steel, and three times tougher than the top quality man-made fiber Kevlar (Gosline, J. M. et al., J. Exp. Biol., 202:3295, 1999; Vollrath, F. & Knight, D. P., Nature 410: 541, 2001). Accordingly, spider dragline silk has received a great deal of attention as a material which can be used in various industrial applications. Also, spider dragline silk is biocompatible and biodegradable, and thus is envisioned in many biomedical applications. Unfortunately, native dragline silk cannot be conveniently obtained by farming spiders, because it is highly territorial and aggressive. Thus, there have been many efforts to produce recombinant dragline silk proteins (Lazaris, A. et al., Science, 295:472, 2002; Teule, F. et al., Nat. Protoc., 4: 341, 2009; Arcidiacono, S. et al., Macromolecules, 35: 1262, 2002; Brooks, A. E. et al. Biomacromolecules, 9: 1506, 2008; Heim, M., Keerl, D. & Scheibel, T., Angew. Chem. Int. Ed. Engl., 48: 3584, 2009; Fahnestock, S. R. et al., Rev. Mol. Biotechnol., 74: 105, 2000; Scheller, J. et al., Nat. Biotechnol., 19: 573, 2001; Widmaier, D. M. et al. Mol. Syst. Biol., 5: 309, 2009).

All the spiders studied have evolved dragline silk proteins having a molecular weight of 250-320 kDa. However, the largest of the dragline silk proteins that have been synthesized in *E. coli* has a molecular weight of 163 kDa (Fahnestock S.R. & Irwin S.L., Appl. Microbiol. Biotechnol., 47:23, 1997), which corresponds to half the molecular weight of the dragline silk produced by spiders. The reason why the production of a larger dragline silk protein was not reported is believed to contribute to a problem of non-homogeneity caused by an error occurred during a protein synthesis process.

Accordingly, the present inventors have made many efforts to provide a recombinant dragline silk protein which has a high molecular weight, like a dragline silk protein produced from spiders, and has physical properties similar to or better than native silk protein when being made into a fiber. As a result, the present inventors have found that a spider silk fiber having physical properties better than a conventional native spider silk fiber can be produced by co-expressing glycine tRNA in bacteria such as *E. coli* to produce a recombinant silk protein having a high molecular weight of 284.9 kDa or more and then spinning the recombinant silk protein, thereby completing the present invention.

### DISCLOSURE OF INVENTION

It is an object of the present invention to provide a recombinant silk protein which has a high molecular weight, like native spider dragline silk proteins.

Another object of the present invention is to provide a spider silk fiber having improved physical properties, spun from a high-molecular-weight recombinant silk protein.

In order to accomplish the above objects, the present invention provides a high-molecular-weight recombinant silk or silk-like protein having a structure in which a peptide having a glycine content of 10% or more is repeated 64-160 times.

In addition, the present invention provides a high-molecular-weight recombinant silk protein having a structure in which a peptide of SEQ ID NO: 1 is repeated 64-160 times, the recombinant silk protein having a molecular weight of 192.8-482 kDa.

Also, the present invention provides a method for preparing a high-molecular-weight recombinant silk or silk-like protein, the method comprising co-expressing a gene encoding said recombinant silk or silk-like protein with a nucleotide sequence encoding glycine tRNA.

Further, the present invention provides a method for producing a micro-sized or nano-sized spider silk or spider silk-like fiber, the method comprising spinning a solution containing said high-molecular-weight recombinant silk or silk-like protein.

In addition, the present invention provides a micro-sized or nano-sized spider silk or spider silk-like fiber produced by said method.

In addition, the present invention provides a method for producing a micro-sized or nano-sized spider silk fiber, the method comprising spinning a solution containing a recombinant silk protein, wherein the recombinant silk protein having a structure in which a peptide of SEQ ID NO: 1 is repeated 64-160 times, the recombinant silk protein having a molecular weight of 192.8-482 kDa.

Moreover, the present invention provides a micro-sized or nano-sized spider silk fiber produced by said method.

## BRIEF DESCRIPTION OF THE DRAWINGS

FIG. 1 shows a system for expression of a recombinant silk protein and the amino acid sequence of the repeating unit of the recombinant silk protein.

FIG. 2 is a photograph showing the molecular weights of recombinant silk proteins of 16-mer, 32-mer, 64-mer and 96-mer, separated on 10% SDS-PAGE gel.

FIG. 3 is a graphic diagram showing stress-strain curves of fibers spun from 20% (w/v) recombinant silk protein solutions using a wet spinning method.

FIG. 4 is a graphic diagram showing the breaking strain, tenacity and Young's modulus of fibers spun from 20% (w/v) recombinant silk protein solutions using a wet spinning method.

FIG. 5 is a graphic diagram showing the breaking strain, tenacity and Young's modulus of fibers as function of the recombinant silk protein concentrations of dope solutions.

## BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods are those well known and commonly employed in the art.

The definition of main terms used in the detailed description of the invention is as follows.

As used herein, the term "silk protein" refers to a synthetic silk protein that approximates the molecular and structural profile of native silk proteins and is biosynthesized by a recombinant protein production method. Examples of the silk protein include dragline silk, silk fibroin and flagelliform silk proteins. As used herein, the term "silk-like protein" refers to a protein which comprises as a repeating unit a peptide having a glycine content of 10% or more and is produced by, for example, a recombinant protein production method, like the silk protein. Examples of the silk-like protein include elastin, byssus, and collagen.

As used herein, the term "spider silk fiber" refers to a fiber which is produced from a synthetic recombinant silk protein and is substantially similar to a native spider silk fiber. The term "spider silk-like fiber" refers to a fiber which is produced from a synthetic recombinant silk-like protein and has physical properties similar to a spider silk protein.

As used herein, the term "recombinant protein" refers to a protein produced by expression of a nucleic acid sequence which is incorporated into a vector, i.e., e.g., an autonomously replicating plasmid or virus, or into the genomic DNA of a host cell, or which exists as a separate molecule in the host cell.

As used herein, the term "host cell" refers to any cell capable of expressing a functional gene and/or gene product introduced from another cell or organism.

In one aspect, the present invention is directed to a high-molecular-weight recombinant silk or silk-like protein having a structure in which a peptide having a glycine content of 10% or more is repeated 64-160 times.

In the present invention, the peptide having a glycine content of 10% or more, which constitutes the silk protein or silk-like protein, is preferably a repeating peptide constituting a protein selected from the group consisting of dragline silk protein, elastin, silk fibroin, byssus, flagelliform silk protein and collagen. Amino acid sequences of SEQ ID NOS: 1 to 4 are repeating peptides of dragline silk protein, amino acid sequences of SEQ ID NOS: 5-7 are repeating peptides of eastin, an amino acid sequence of SEQ ID NO: 8 is a repeating peptide of silk fibroin, an amino acid sequence of SEQ ID NO: 9 is a repeating peptide of byssus, and an amino acid sequence of SEQ ID NO: 10 is a repeating peptide of flagelliform silk protein, and amino acid sequences of SEQ ID NOS: 11 and 12 are repeating peptides of collagen.

SEQ ID NO: 1: $NH_2$-SGRGGLGGQGAGMAAAAAMGGAGQGGYGGLGSQGT-COOH
SEQ ID NO: 2: $NH_2$-GPGQQ-COOH
SEQ ID NO: 3: $NH_2$-GPGGY-COOH
SEQ ID NO: 4: $NH_2$-GGYGPGS-COOH
SEQ ID NO: 5: $NH_2$-GVGVP-COOH
SEQ ID NO: 6: $NH_2$-VPGG-COOH
SEQ ID NO: 7: $NH_2$-APGVGV-COOH
SEQ ID NO: 8: $NH_2$-GAGAGS-COOH
SEQ ID NO: 9: $NH_2$-GPGGG-COOH
SEQ ID NO: 10: $NH_2$-GPGGX-COOH
SEQ ID NO: 11: $NH_2$-GAPGAPGSQGAPGLQ-COOH
SEQ ID NO: 12: $NH_2$-GAPGTPGPQGLPGSP-COOH

In the present invention, it was demonstrated that a recombinant silk protein prepared to comprise 64 repeats of the amino acid sequence set forth in SEQ ID NO: 1 (hereinafter referred to as "64-mer") has a molecular weight of about 192.8 kDa, thus making it possible to produce a recombinant silk protein having a higher molecular weight than the existing largest dragline silk protein (163 kDa) synthesized in *E. coli.* In addition, it was found that a recombinant silk protein prepared to comprise 96 repeats of the amino acid sequence set forth in SEQ ID NO: 1 (hereinafter referred to as "96-mer") has a molecular weight reaching 284.9 kDa which is substantially similar to the molecular weight of native silk proteins (250-320 kDa) obtained from spiders.

However, recombinant silk proteins comprising 160 repeats or more of the peptide sequence cannot be synthesized by *E. coli.* Thus, the recombinant silk or silk-like protein according to the present invention preferably has a structure in which the peptide is preferably repeated 64-160 times, more preferably 80-160 times, and even more preferably 96-160 times.

In the present invention, the amino acid sequences that are used as repeating units are not limited to the exact sequences of SEQ ID NOS: 1 to 12. The amino acid sequences indicated herein also comprise variants. Thus, the amino acid sequences of the proteins of the present invention also encompass all sequences differing from the herein-disclosed sequences by amino acid insertions, deletions, and substitutions.

Preferably, amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" in the repeating unit are typically in the range of about 1 to 5 amino acids, and preferably about 1, 2 or 3 amino acids. Amino acid additions in the repeating unit are typically less than 100, preferably less than 80, more preferably less than 50, most preferably less than 20 amino acids, which are inserted into the repeating unit of the present invention and added on and/or inserted into the protein of the present invention. It is noted that only those additions are contemplated in the present invention, which do not negatively affect the characteristics of the protein disclosed herein.

The variation allowed may be experimentally determined by systematically making insertions, deletions, or substitutions of amino acids in a protein using recombinant DNA techniques and assaying the resulting recombinant variants for activity. This does not inquire more than routine experiments for a person skilled in the art.

Accordingly, the protein of the present invention comprises as a repeating unit an amino acid sequence having a homology of at least 90% with SEQ ID NO: 1. As used herein, the phrase "having a homology of at least 90%" means that the protein has an identity of 91, 91.5, 92, 92.5, 93, 93.5, 94, 94.5, 95, 95.5, 96, 96.5, 97, 97.5, 98, 98.5, 99 or 99.5% with the sequence of SEQ ID NO: 1. The term "homology" refers to the degree of similarity between two amino acid sequences. Homologous proteins are those that are similar in sequence and function. Homology comparisons can be conducted by eye, or more usually, with the aid of readily available sequence comparison programs. These commercially available computer programs can calculate percent homology between two or more sequences (Wilbur, W. J. & Lipman, D. J., Proc. Natl. Acad. Sd. USA., 80:726, 1983).

In the present invention, the recombinant silk protein or the recombinant silk-like protein is preferably prepared by co-expressing glycine tRNA in bacteria. Accordingly, in another aspect, the present invention relates to a method for preparing the recombinant silk or silk-like protein, the method comprising co-expressing a gene encoding the recombinant silk protein or silk-like protein with a nucleotide sequence encoding glycine tRNA. Herein, the bacteria may be *E. coli.* Namely, the preparation of high-molecular-weight silk proteins has not been reported in the prior art; however, in the present invention, a recombinant silk protein having a high molecular weight of 192.8 kDa or more was prepared by co-expressing a gene encoding a silk protein, having as a repeating unit an amino acid sequence of SEQ ID NO: 1, with a nucleotide sequence encoding glycine tRNA in bacteria such as *E. coli.* Thus, the inventive silk protein having as a repeating unit the amino acid sequence of SEQ ID NO: 1 is characterized in that it has a molecular weight of 192.8-482 kDa.

In another aspect, the present invention is directed to a spider silk fiber or spider silk-like fiber having improved physical properties, produced by spinning the high-molecular-weight recombinant silk protein or silk-like protein.

In the present invention, a micro-sized or nano-sized spider silk fiber can be produced by spinning a dope solution containing the high-molecular-weight recombinant silk protein or silk-like protein through a spinneret.

As used herein, the term "dope solution" refers to any liquid mixture that contains silk protein and is amenable to extrusion for the formation of a spider silk fiber or film casting. Dope solutions may also contain, in addition to protein monomers, higher order aggregates including, for example, dimers, trimers, and tetramers. Normally, dope solutions are aqueous solutions of pH 4.0-12.0 and have less than 40% (w/v) organics or chaotropic agents. Preferably, the dope solution does not contain any organic solvents or chaotropic agents, but may include additives to enhance preservation, stability, or workability of the solution.

In the present invention, the dope solution preferably comprises 20-80% (w/v) of a recombinant silk protein.

In addition, the dope solution is preferably wet-spun in a liquid bath. Preferably, the liquid bath contains a liquid selected from the group consisting of methanol, ethanol, isopropanol, acetonitrile, water and aqueous ammonium sulfate.

Meanwhile, the diameter of the spider silk fiber can be determined by the diameter of the spinneret. The diameter of the spider silk fiber may be, for example, 0.6~50 μm, but the scope of the present invention is not limited thereto.

In one Example of the present invention, the physical properties (e.g., tenacity, Young's modulus and breaking strain) of the spider silk fiber produced by wet spinning were measured. The measurement results indicated that a spider silk fiber having improved physical properties can be provided using the recombinant silk protein according to the present invention. Particularly, a fiber produced from a recombinant silk protein of 96-mer showed a tenacity of 508 ± 108 MPa and a breaking strain of 15 ± 5%, which are comparable to the values reported for native *N. clavipes* dragline silk (740-1,200 MPa and 18-27%). In particular, the Young's modulus of the 96-mer fiber was 21±4 GPa corresponding to twice that of the native dragline silk (11-14 GPa). The tenacity of the 96-mer fiber according to the present invention was 508 ± 108 MPa which is the highest ever reported for recombinant spider silk proteins.

In addition to the above-described wet-spinning method, the fiber can also be produced by an electrical spinning method in which voltage is applied to a solution containing the high-molecular-weight recombinant silk or recombinant silk-like protein so that the solution is extruded in the direction of the applied electric field. According this method, a micro-sized or nano-sized spider silk fiber or spider silk-like fiber can be obtained.

For example, a 12% (w/v) silk protein solution can be obtained by dissolving the recombinant silk protein in hexafluoroisopropanol (HFIP; Sigma) at room temperature for 2 days. In an electrical spinning process, a voltage of 12 kV is applied to one silk solution drop at the tip of a glass pipette including platinum wire electrodes, and when the applied electric force exceeds the surface tension of the silk solution drop, a fiber jet is formed and extruded in the direction of the applied electric field. The fiber can be collected on a glass material formed on a flat plate covered with aluminum, and the electrospun fiber may be treated with methanol to induce formation of the beta plane. The fiber may be allowed to stand at room temperature for 24 hours and then dried in air for 3 days, after which the physical properties thereof can be measured.

Next, from atomic force microscopy force curve measurements, the Young's modulus of each specimen used can be calculated. The sensitivity of the photodetector for conversion of the deflection of the cantilever is measured at room temperature (21 °C), a force-distance curve is plotted using an atomic force microscope (Dimension V; Veeco Instruments Inc., Plainview, NY) together with a silicon cantilever. 20 measurements are made for each specimen, and for the measurement of Young's modulus, a modified Hertz model can be applied to each force curve (Hertz, 1882, Sneddon, 1965). The Young's modulus of each specimen is calculated directly from the obtained parameters. In the Hertz model, Young's model (*E*) is given by the following equations:

$$E = \mathrm{p}F(1-v^2)/\ (2a^2\mathrm{tana})\quad (1)$$

$$F = kd\quad (2)$$

wherein *F, v, a, k* and *d* indicate the force at the tip, the Poisson's ratio, the strain of the specimen, the spring constant of the cantilever, and the deflection of the cantilever, respectively. From data, including the shape and spring constant of the tip, the kind and deflection of the cantilever, and the Poisson's ratio of the specimen, the Young's modulus of the fiber can be determined.

Meanwhile, the recombinant silk protein/recombinant silk-like protein as defined herein and a fiber, filament, film, foam, sphere, nanofibril, hydrogen and the like produced therefrom may be used in the field of biotechnology and/or medicine, preferably for the manufacture of wound closure or coverage systems, suture materials for use in neurosurgery or ophthalmic surgery. Furthermore, the protein/thread may preferably be used for the manufacture of replacement materials, preferably artificial cartilage or tendon materials.

Additionally, the spider silk fiber or spider silk-like fiber of the present invention can be used in the manufacture of medical devices such as medical adhesive strips, skin grafts, replacement ligaments, and surgical mesh; and in a wide range of industrial and commercial products, such as clothing fabric, bullet-proof vest lining, container fabric, bag or purse straps, cable, rope, adhesive binding material, non-adhesive binding material, strapping material, automotive covers and parts, aircraft construction material, weatherproofing material, flexible partition material, sports equipment; and, in fact, in nearly any use of fibrils or fabric for which high tensile strength and elasticity are desired characteristics. Adaptability and use of the stable fibril product in other forms, such as a dry spray coating, bead-like particles, or use in a mixture with other compositions is also contemplated by the present invention.

The recombinant silk protein or recombinant silk-like protein of the present invention may be added to cellulose and keratin and collagen products and thus, the present invention is also directed to a paper or a skin care and hair care product, comprising cellulose and/or keratin and/or collagen and the recombinant protein of the present invention. Papers and skin care and hair care products, in which the proteins of the present invention are incorporated, show improved characteristics, in particular improved tensile strength or tear strength. Furthermore, the high-molecular-weight recombinant protein of the present invention may be used as a coating for textile and leather products, thereby conferring stability and durability to the coated product. The silk protein in particular show applicability for coating leather products, since in this case, tanning and its negative effects for environment can be avoided or at least reduced.

**EXAMPLES**

Hereinafter, the present invention will be described in further detail with reference to examples. It will be obvious to a person having ordinary skill in the art that these examples are illustrative purposes only and are not to be construed to limit the scope of the present invention.

**Example 1: Construction of vectors pSH32, pSH48, pSH64, pSH80 and pSH96 for expression of high-molecular-weight silk proteins and vector for expression of nucleotide sequence encoding glycine tRNA and preparation of silk proteins having various molecular weights**

1-1: Construction of pSH32, pSH48, pSH64, pSH80 and pSH96

All procedures for genetic manipulation were carried out according to standard methods (Sambrook et al., Molecular cloning: a laboratory manual, 2nd Ed., Cold Spring Harbor Laboratory Press, Cold Spring Harbor, New York, 1989). In order to construct the recombinant plasmid pSH32, the plasmid pSH16a (Lee et al., Theories and Applications of Chem. Eng., 8(2):3969, 2002) (SEQ ID NO: 13) was digested with the restriction enzymes *Spe*I and *Nhe*I (New England Biolabs, USA) to obtain a 1.7-kb fragment which was then treated with the restriction enzyme *Spe*I and ligated to the dephosphorylated plasmid pSH16a, thereby obtaining the recombinant plasmid pSH32 comprising a nucleic acid sequence encoding a 32-mer silk protein of SEQ ID NO: 14. The orientation of the insert was checked by digestion with the restriction enzymes *Spe*I and *Nhe*I. In the same manner, the plasmid pSH16a was digested with the restriction enzymes *Spe*I and *Nhe*I to obtain a 1.7-kb fragment which was then ligated to the plasmid pSH32 digested with the restriction enzyme *Spe*I, thereby obtaining the recombinant plasmid pSH48. Also, the plasmid pSH32 was digested with the restriction enzymes *Spe*I and *Nhe*I to obtain a 3.4-kb fragment which was then ligated to the plasmid pSH32 digested with the restriction enzyme *Spe*I, thereby obtaining the recombinant plasmid pSH64 comprising a nucleic acid sequence encoding a 64-mer silk protein of SEQ ID NO: 15. The orientation of each insert was checked by digestion with the restriction enzymes *Spe*I and *Nhe*I. In addition, in the same manner, the DNA fragment of each of the plasmids pSH16a and pSH32 was inserted into the plasmid pSH64 digested with the restriction enzyme *SpeI,* thereby constructing the recombinant plasmid pSH80 comprising a nucleic acid sequence encoding a 80-mer silk protein of SEQ ID NO: 16, and the plasmid pSH96 comprising a nucleic acid sequence encoding a 96-mer silk protein of SEQ ID NO: 17. The orientation of the insert in each of the plasmids was checked by digestion with the restriction enzymes *Spe*I and *Nhe*I. FIG. 1 shows a structure for expression of the recombinant silk protein and the amino acid sequence of the repeating unit of the protein. In this regard, a nucleic acid sequence corresponding to the amino acid repeating unit of SEQ ID NO: 1 is set forth in SEQ ID NO: 18.

1-2: Construction of pTet-glyVXY vector

All procedures for genetic manipulation were carried out according to standard methods (Sambrook et al., Molecular cloning: a laboratory manual, 2nd Ed., Cold Spring Harbor Laboratory Press, Cold Spring Harbor, New York, 1989). To obtain a *gly*VWX gene encoding glycine tRNA, PCR was performed using, as a template, a chromosome isolated from *E. coli* W3110 (derived from *E. coli* K-12, $\lambda^-$, $F^-$, prototrophic strain), and primers of SEQ ID NO: 19 and SEQ ID NO: 20.

SEQ ID NO: 19: 5'-GCTCGATATCTAACGACGCAGAAATGCGAAA-3'

SEQ ID NO: 20: 5'-CATTGGATCCTAAGATTACAGCCTGAGGCTGTG-3'

The PCR reaction was performed using *Pfu* polymerase (SolGent, Korea) under the following conditions: initial denaturation at 95 °C for 4 min; then 10 cycles of denaturaion at 95 °C for 20 sec, annealing at 51 °C for 30 sec, and extension at 72 °C for 60 sec; and then 19 cycles of denaturation at 95 °C for 20 sec, annealing at 60 °C for 30 sec, and extension at 72 °C for 60 sec; followed by final extension at 72 °C for 5 min.

The PCR product DNA was electrophoresed on agarose gel to obtain a purified 479-bp PCR product. The PCR product was digested with the restriction enzymes *Bam*HI and *Eco*RV (New England Biolabs, USA), and in order to use the promoter of a tetracycline resistant gene (*tet*) which can be continually used, the plasmid was also digested with the same restriction enzymes. The digested PCR product and plasmid T4 DNA were ligated with each other by ligase (Roche, Germany), and the ligated product was transformed into *E. coli* Top10 ($F^-$ *mcrA Δ*(*mrr-hsd*RMS-*mcr*BC) ₵0*lac*ZΔM15 *Δlac*X74 *rec*A1 *ara*D139 Δ(*araleu*)*7697 galU galK* rpsL ($Str^R$) *end*A1 *nup*G). The transformed strain was selected on LB agar solid medium (10 g/L tryptone, 5 g/L yeast extract, 5 g/L NaCl, and 15 g/L agar) containing 34 mg/L chloramphenicol, thereby constructing the recombinant plasmid pTet-glyVXY. The constructed recombinant plasmid was confirmed by digestion with restriction enzymes and base sequence analysis.

1-3: Construction of recombinant plasmid pTet-gly2

All procedures for genetic manipulation were carried out according to standard methods (Sambrook et al., Molecular cloning: a laboratory manual, 2nd Ed., Cold Spring Harbor Laboratory Press, Cold Spring Harbor, New York, 1989). To further express the *gly*VXY gene encoding glycine tRNA, PCR was performed using pTet-glyVXY as a template and primers of SEQ ID NOS: 21 and 22.

SEQ ID NO: 21: 5'-GGCTCGCATGCTCATGTTTGACAGCTTATCATCGA-3'

SEQ ID NO: 22: 5'-ATTGTCGACTGCTGCAGTAAGATTACAGCCTGAGGCTGTG-3'

The PCR reaction was performed using *Pfu* polymerase (SolGent, Korea) under the following conditions: initial denaturation at 95 °C for 3 min; then 10 cycles of denaturation at 95 °C for 20 sec, annealing at 52 °C for 30 sec, and extension at 72 °C for 50 sec; and then 19 cycles of denaturation at 95 °C for 20 sec, annealing at 62 °C for 30 sec, and extension at 72 °C for 50 sec; followed by final extension at 72 °C for 5 min.

The DNA obtained by the PCR reaction was electrophoresed on agarose gel to obtain a purified 674-bp PCR product. The 647-bp PCR product and the plasmid pTet-glyVXY were digested with the restriction enzymes *Sph*I and *Sal*I (New England Biolabs, USA) and ligated with each other by T4 DNA ligase (Roche, Germany), and the ligated product was transformed into *E. coli* Top10. The transformed strain was selected on LB agar solid medium (10 g/L tryptone, 5 g/L yeast extract, 5 g/L NaCl, and 15 g/L agar) containing 34 mg/L chloramphenicol, thereby constructing the recombinant plasmid pTet-gly2. The constructed recombinant plasmid was confirmed by digestion with restriction enzymes and base sequence analysis.

1-4: Preparation of recombinant silk proteins having various molecular weights

In order to prepare silk proteins having various molecular weights, each of the pSH16a vector and pSH32 vector constructed in Example 1-1 was introduced into the recombinant plasmid pTet-glyVXY, obtained in Example 1-2, and *E. coli* BL21 (DE3) *(F- ompT hsd*SB(*rB- mB-) gal dcm* (DE3); a prophage carrying the T7 RNA polymerase gene) (New England Biolabs, USA). Meanwhile, each of the pSH64 vector and the pSH96 vector was introduced into the pTetgly2 vector, obtained in Example 1-3, and *E. coli* BL21 (DE3). The strains thus transformed were inoculated into LB liquid medium (10 g/L tryptone, 5 g/L yeast extract, and 5 g/L NaCl) containing 34 mg/L chloramphenicol and 25 mg/L kanamycin and were cultured with continuous shaking at 30 °C at 180 rpm. When the optical density (O.D.) measured with a spectrophotometer at a wavelength of 600 nm after inoculation of 1% of each strain reached 0.2, 0.4 or 0.6, 1 mM IPTG was added to each strain to induce the expression of silk proteins. 5 hours after induction of the expression of the silk proteins, the cultures were harvested.

For analysis of the prepared recombinant proteins, each of the harvested cultures was centrifuged at 4 °C at 10,000 g for 10 minutes to obtain cell pellets which were then dissolved in TE buffer and 5x Laemmli sample buffer. The same amount (0.024 mg) of samples were taken from the cultures using 10% SDS-PAGE and stained with Coomassie brilliant blue R250 (Bio-Rad, USA), followed by quantification with GS-710 Calibrated Imaging Densitometer (Bio-Rad, USA). The protein contents of the samples were measured by the Bradford assay using bovine serum albumin as a standard (Bradford, M. M., Anal. Biochem., 72:248, 1976).

As a result, as shown in FIG. 2, the recombinant silk proteins of 16-mer (prepared using the pSH16a vector), 32-mer (prepared using the pSH32 vector), 64-mer (prepared using the pSH64 vector) and 96-mer (prepared using the pSH96) had molecular weights of about 50.4 kDa, 100.7 kDa, 192.8 kDa and 284.9 kDa, respectively. In the prior art, it has been known that the largest of the dragline silk proteins that have been synthesized in *E. coli* has a molecular weight of 163 kDa and that it is difficult to produce a silk protein having a molecular weight larger than 163 kDa (Fahnestock, S. R. & Irwin, S. L., Appl. Microbiol. Biotechnol., 47:23, 1997; Vendrely, C. & Scheibel, T., Macromol. Biosci., 7:401, 2007; Lazaris, A., et al., Science, 295:472, 2002). However, according to the present invention, it was found that recombinant silk proteins having high molecular weights, such as 192.8 kDa and 284.9 kDa, can be provided by co-expressing the glycine tRNA-encoding nucleotide sequence with the expression vector as described above.

**Example 2: Production of spider silk fiber by wet-spinning method - effect of molecular weight on mechanical properties of wet-spun fiber**

Each of the recombinant silk proteins prepared in Example 1-2 was dissolved in hexafluoroisopropanol (HFIP; Sigma), a spinning solvent, thus preparing spinning dope solutions. Each of the dope solutions was extruded using a pump (KDS100; KD Scientific) at a rate of 1-2 ml/hr. With the silk protein concentration of the dope solutions, all the silk proteins were spun at a spider silk protein concentration of 20% (w/v), which was the maximum operational concentration for the native-sized 96-mer protein due to the solubility and viscosity. At this time, each dope solution was extruded from a 1-ml Kovax syringe through a 26-G syringe needle (Korea Vaccine Co., Ltd.) into a solidification bath containing 90% (v/v) methanol.

After the spinning process, each of the spun fibers was allowed to stand in the solidification bath for 20 minutes and was hand-drawn up to 5 times the original length. FIG. 3 shows stress-strain curves of the fibers.

Next, the fibers were dried at room temperature and continuously maintained under tension in order to prevent shrinkage and maintain the stretched lengths of the fibers during measurement.

Before the test, specimens (n=10) of the fibers were conditioned at room temperature at a relative humidity of 50% for 24 hours. The tensile test was performed with a universal tensile tester (RB302 ML, R&B Inc.) using a 100 g load cell. The gauge length was 20 mm, and the cross-head speed was 10 mm/min. Mechanical properties data are shown as means $\pm$ standard deviation (n=10). Statistical analysis was performed by unpaired t-test, and $P < 0.05$ was considered statistically significant.

The measurement results are shown in FIG. 4. As can be seen therein, as the molecular weights of the silk proteins increased, the mechanical properties (such as breaking strain, tenacity and Young's modulus) of the fibers were improved (32-mer: breaking strain of 3.27$\pm$0.32%, tenacity of 202$\pm$25 MPa, and Young's modulus of 8.28$\pm$0.85 GPa; 64-mer: breaking strain of 4.31$\pm$0.64%, tenacity of 252$\pm$26 MPa, and Young's modulus of 10.14$\pm$0.67 GPa; and 96-mer: breaking strain of 15$\pm$5%, tenacity of 508$\pm$108 MPa, and Young's modulus of 21$\pm$4 GPa). Particularly, the spider silk fiber produced from the recombinant silk protein of 96-mer having a molecular weight reaching 284.9 kDa showed unexpected significant improvements in all breaking strain, tenacity and Young's modulus compared to the spider silk fibers produced from the recombinant silk proteins of 64-mer or less.

Specifically, the fiber spun from the recombinant silk protein of 96-mer showed a tenacity of 508 $\pm$ 108 MPa and a breaking strain of and 15 $\pm$ 5%, which are comparable to those of native *N. clavipes* dragline silk (740-1,200 MPa; 18-27%). Particularly, the Young's modulus of the 96-mer fiber was 21 $\pm$ 4 GPa corresponding to twice that of the native dragline silk (11-14 GPa). The tenacity of the 96-mer fiber (508-108 MPa) in the present invention is the highest ever reported for recombinant spider silk proteins.

**Example 3: Effect of recombinant silk protein concentrations of dope solutions on properties of wet-spun fibers**

In order to examine the effects of the recombinant silk protein concentrations of dope solutions on the properties of spun fibers, each of the 16-mer, 32-mer and 64-mer proteins was spun at the maximum operational concentrations, and test results for the spun fibers were compared with the results of Example 2.

As a result, as shown in FIG. 5, when the concentration of the 16-mer protein was increased from 20% to the maximum concentration of 30%, the properties of the fiber spun from the protein were significantly improved. However, when the concentration of the 32-mer protein was increased from 20% to the maximum concentration of 27%, the breaking strain and tenacity of the fiber were increased, but these increases were not statistically significant. In addition, the maximum operational concentration of the 64-mer protein was 23%, the mechanical properties of the fiber spun from the dope solution having the 64-mer protein concentration of 23% did not significantly differ from those of the fiber spun from the dope solution having the 64-mer protein concentration of 20%.

**INDUSTRIAL APPLICABILITY**

The present invention relates to a high-molecular-weight recombinant silk or silk-like protein having a molecular weight which is substantially similar to that of native silk protein, and to a micro- or nano-sized spider silk or silk-like fiber having improved physical properties, produced therefrom. The recombinant silk or silk-like protein according to the invention has high molecular weight, like dragline silk proteins from spiders, while a fiber produced therefrom has excellent physical properties compared to a fiber produced from native silk protein. Thus, the recombinant silk or silk-like protein and the spider silk or silk-like fiber produced therefrom will be highly useful in various industrial applications, including bioengineering applications and medical applications.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims

and equivalents thereof.

## Claims

1. A high-molecular-weight recombinant silk or silk-like protein having a structure in which a peptide having a glycine content of 10% or more is repeated 64-160 times.

2. The high-molecular-weight recombinant silk or silk-like protein according to claim 1, having a structure in which the peptide is repeated 80-160 times.

3. The high-molecular-weight recombinant silk or silk-like protein according to claim 1, having a structure in which the peptide is repeated 96-160 times.

4. The high-molecular-weight recombinant silk or silk-like protein according to claim 1, wherein the peptide is a repeating peptide constituting a protein selected from the group consisting of dragline silk, elastin, silk fibroin, byssus, flagelliform silk and collagen.

5. The high-molecular-weight recombinant silk or silk-like protein according to claim 1, wherein the peptide has one of the amino acid sequences of SEQ ID NO: 1 to 11.

6. The high-molecular-weight recombinant silk or silk-like protein according to claim 5, repeating an amino acid sequence having a homology of at least 90% with the peptide.

7. A high-molecular-weight recombinant silk protein having a structure in which a peptide of SEQ ID NO: 1 is repeated 64-160 times, and having a molecular weight of 192.8-482 kDa.

8. A method for preparing a high-molecular-weight recombinant silk or silk-like protein, comprising co-expressing a gene encoding recombinant silk or silk-like protein according to one of claims 1 to 7 with a nucleotide sequence encoding glycine tRNA in bacteria.

9. The method according to claim 8, wherein the bacteria is *E. coli* .

10. A method for preparing a micro-sized or nano-sized spider silk or spider silk-like fiber, comprising spinning a dope solution containing the high-molecular-weight recombinant silk or silk-like protein according to one of claims 1 to 6.

**11.** The method according to claim 10, comprising wet-spinning a dope solution containing 20-80% (w/v) of a recombinant silk or silk-like protein.

**12.** The method according to claim 11, wherein the dope solution is spun in a liquid bath.

**13.** The method according to claim 12, wherein the liquid bath contains a liquid selected from the group consisting of methanol, ethanol, isopropanol, acetonitrile, water and aqueous ammonium sulfate.

**14.** A micro-sized or nano-sized spider silk or spider silk-like fiber prepared by the method according to claim 10.

**15.** A method for preparing a micro-sized or nano-sized spider silk fiber, comprising spinning a dope solution containing the high-molecular-weight recombinant silk protein according to claim 7.

**16.** A micro-sized or nano-sized spider silk fiber prepared by the method according to claim 15.

**17.** The micro-sized or nano-sized spider silk fiber according to claim 16, wherein the spider silk fiber has at least 252 MPa tenacity, at least 10.14 GPa Young's modulus, and at least 4.31% breaking strain.

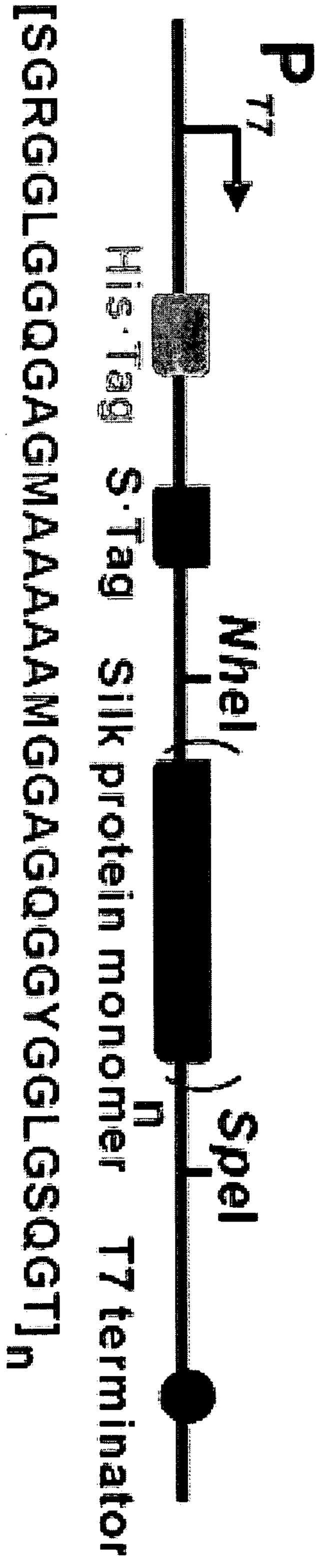
P T7
His-Tag
S-Tag
NheI
Silk protein monomer
n
SpeI
T7 terminator
[SGRGGLGGQGAGMAAAAAMGGAGQGGYGGLGSQGT]n

FIG. 1

FIG. 2

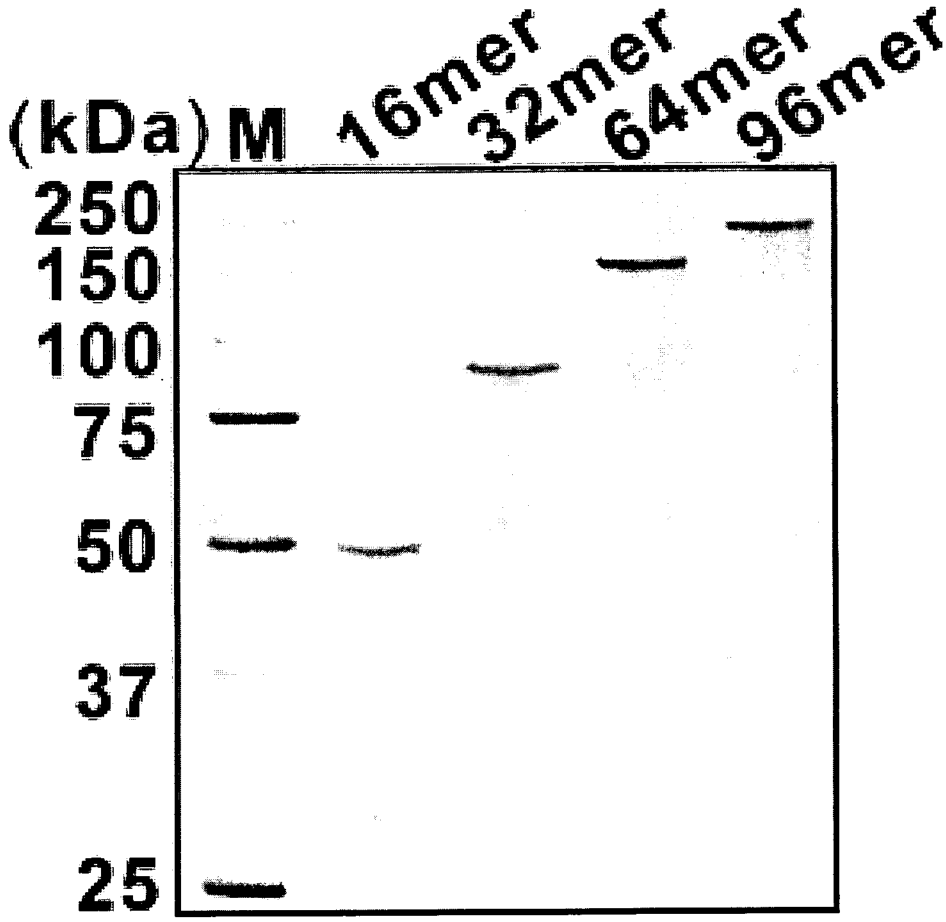

FIG. 3

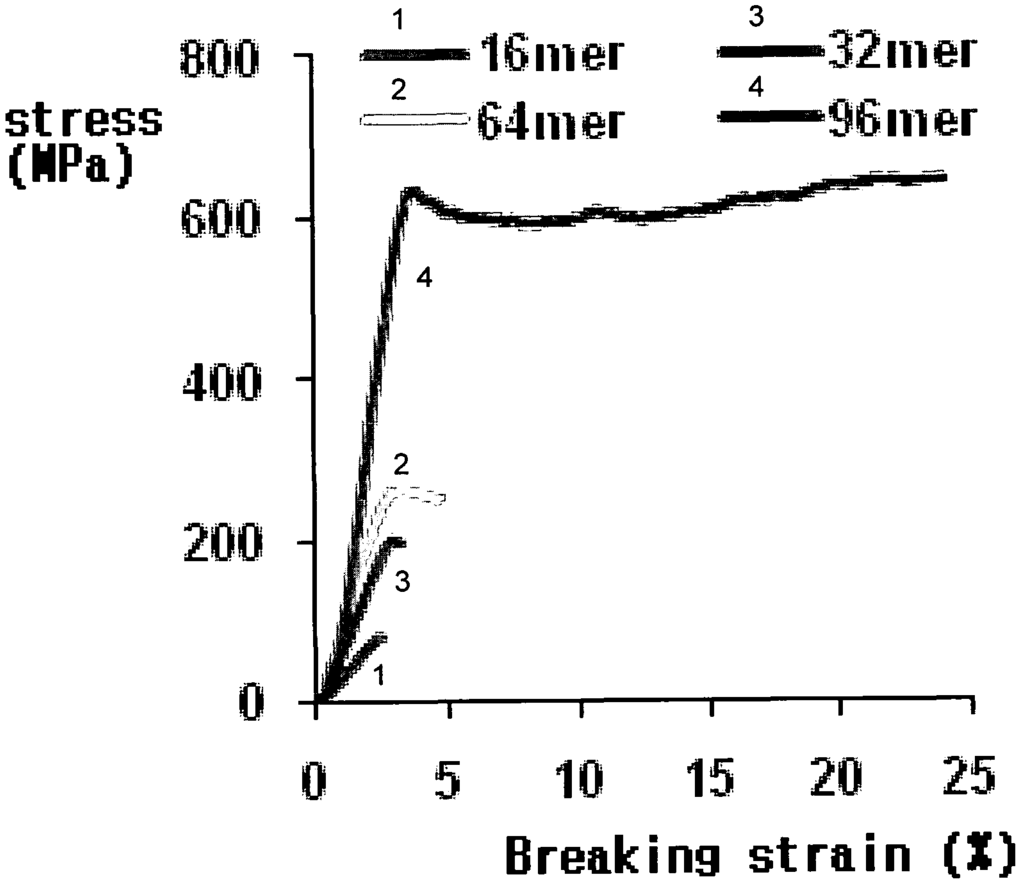
1
16mer
3
32mer
2
64mer
4
96mer
stress
(MPa)
800
600
400
200
0
4
2
3
1
0
5
10
15
20
25
Breaking strain (%)

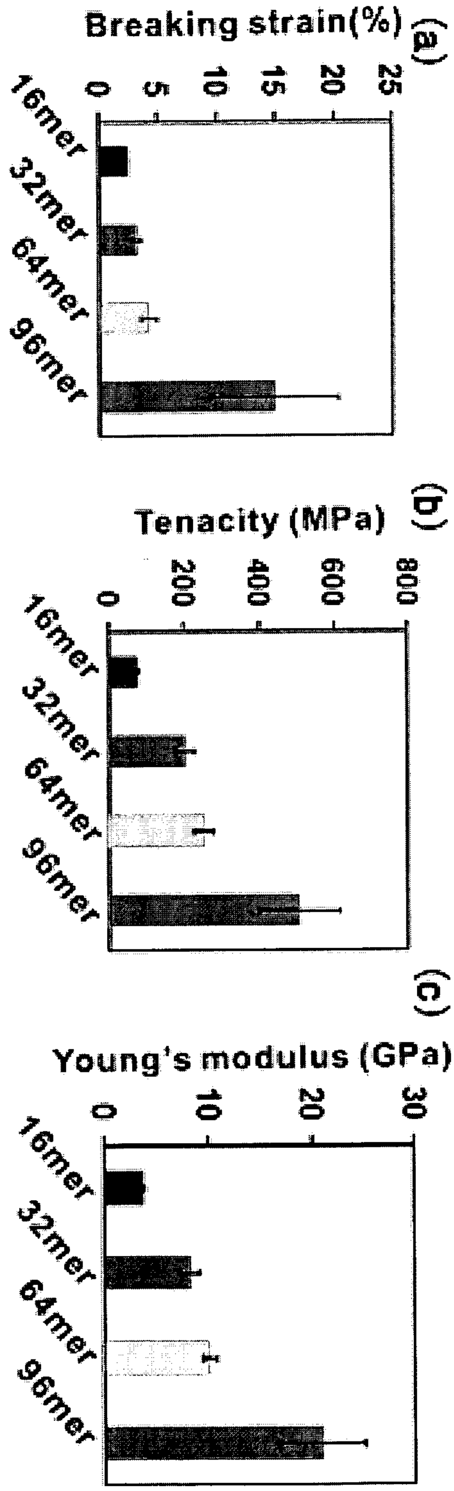
(a)
Breaking strain(%)
0
5
10
15
20
25
16mer
32mer
64mer
96mer
(b)
Tenacity (MPa)
0
200
400
600
800
16mer
32mer
64mer
96mer
(c)
Young's modulus (GPa)
0
10
20
30
16mer
32mer
64mer
96mer

FIG. 4

FIG. 5

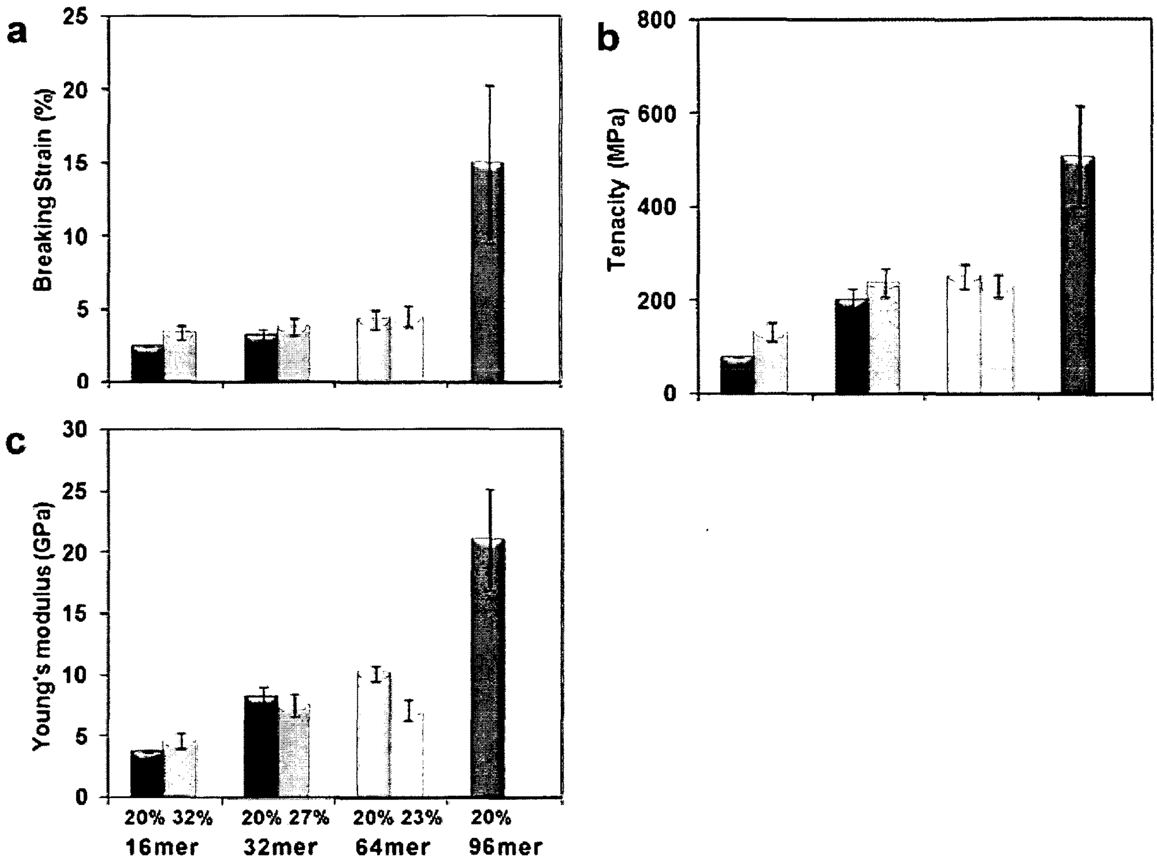
a
b
c
Breaking Strain (%)
Tenacity (MPa)
Young's modulus (GPa)
25
20
15
10
5
0
800
600
400
200
0
30
25
20
15
10
5
0
20% 32%
16mer
20% 27%
32mer
20% 23%
64mer
20%
96mer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GOSLINE, J. M. et al.** *J. Exp. Biol.,* 1999, vol. 202, 3295
- **VOLLRATH, F. ; KNIGHT, D. P.** *Nature,* 2001, vol. 410, 541
- **LAZARIS, A. et al.** *Science,* 2002, vol. 295, 472
- **TEULE, F. et al.** *Nat. Protoc.,* 2009, vol. 4, 341
- **ARCIDIACONO, S. et al.** *Macromolecules,* 2002, vol. 35, 1262
- **BROOKS, A. E. et al.** *Biomacromolecules,* 2008, vol. 9, 1506
- **HEIM, M. ; KEERL, D. ; SCHEIBEL, T.** *Angew. Chem. Int. Ed. Engl.,* 2009, vol. 48, 3584
- **FAHNESTOCK, S. R. et al.** *Rev. Mol. Biotechnol.,* 2000, vol. 74, 105
- **SCHELLER, J. et al.** *Nat. Biotechnol.,* 2001, vol. 19, 573
- **WIDMAIER, D. M. et al.** *Mol. Syst. Biol.,* 2009, vol. 5, 309
- **FAHNESTOCK S.R. ; IRWIN S.L.** *Appl. Microbiol. Biotechnol.,* 1997, vol. 47, 23
- **WILBUR, W. J. ; LIPMAN, D. J.** *Proc. Natl. Acad. Sd. USA.,* 1983, vol. 80, 726
- **SAMBROOK et al.** Molecular cloning: a laboratory manual. Cold Spring Harbor Laboratory Press, 1989
- **LEE et al.** *Theories and Applications of Chem. Eng.,* 2002, vol. 8 (2), 3969
- **BRADFORD, M. M.** *Anal. Biochem.,* 1976, vol. 72, 248
- **FAHNESTOCK, S. R. ; IRWIN, S. L.** *Appl. Microbiol. Biotechnol.,* 1997, vol. 47, 23
- **VENDRELY, C. ; SCHEIBEL, T.** *Macromol. Biosci.,* 2007, vol. 7, 401